# FASCICULE DE BREVET EUROPEEN

(11) **EP 0 740 931 B1**
(45) Date de publication et mention de la délivrance du brevet: **20.08.1997**
(21) Numéro de dépôt: 96400950.0
(22) Date de dépôt: 03.05.1996
(51) Int. Cl.: A61K 7/13, C07D 231/38, B65D 81/32, A61K 7/00

(54) **Compositions pour la teinture des fibres kératiniques contenant des diamino pyrazoles, procédé de teinture, nouveaux diamino pyrazoles et leur procédé de préparation**
Zusammensetzungen für die Färbung von keratinischen Fibern, die Diaminopyrazole enthalten, Verfahren zum Färben, Diaminopyrazole und Verfahren zu ihrer Herstellung
Compositions for dyeing keratinic fibers containing diaminopyrazoles, process for dyeing, diaminopyrazoles and process for their preparation

(30) Priorité: 05.05.1995 FR 9505422
(43) Date de publication de la demande: 06.11.1996
(73) Titulaire: L'OREAL, 75008 Paris (FR)
(72) Inventeur: Vidal, Laurent, 75013 Paris (FR); Burande, Agnès, 77270 Villeparisis (FR); Malle, Gérard, 77100 Meaux (FR); Hocquaux, Michel, 75012 Paris (FR)
(74) Mandataire: Miszputen, Laurent

(56) Documents cités:
- EP-A- 0 216 334
- EP-A- 0 375 977
- WO-A-94/08969
- WO-A-94/08970
- WO-A-94/08971
- GB-A- 2 180 215

## Description

L'invention a pour objet de nouvelles compositions pour la teinture d'oxydation des fibres kératiniques comprenant au moins un 4,5-diamino pyrazole 3-substitué à titre de base d'oxydation, le procédé de teinture mettant en oeuvre cette composition, de nouveaux 4,5-diamino pyrazoles 3-substitués ainsi que leur procédé de préparation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Pour obtenir des nuances rouges, on utilise habituellement, seul ou en mélange avec d'autres bases, et en association avec des coupleurs appropriés, du para-aminophénol, et pour obtenir des nuances bleues, on fait généralement appel à des paraphénylènediamines.

Il a déjà été proposé, notamment dans la demande de brevet EP-A-375 977, d'utiliser certains dérivés de diamino pyrazoles, à savoir plus précisément des 3,4- ou des 4,5-diamino pyrazoles, pour la teinture d'oxydation des fibres kératiniques dans des nuances rouges. Toutefois, l'utilisation des diamino pyrazoles décrits dans cette demande de brevet ne permet pas d'obtenir une riche palette de couleurs et, de plus, le procédé de préparation de ces composés est long et coûteux.

Or, la demanderesse vient maintenant de découvrir, de façon totalement inattendue et surprenante, que l'utilisation de certains 4,5-diamino pyrazoles 3-substitués de formule (I) définie ci-après, pour partie nouveaux en soi, non seulement convient pour une utilisation comme précurseurs de colorant d'oxydation, mais en outre qu'ils permettent d'obtenir des compositions tinctoriales conduisant à des colorations puissantes, dans des nuances allant du rouge jusqu'au bleu. Enfin, ces composés s'avèrent être aisément synthétisables.

Ces découvertes sont à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture, au moins un 4,5-diamino pyrazole 3-substitué de formule (I) ci-dessous à titre de base d'oxydation et/ou au moins un de ses sels d'addition avec un acide : 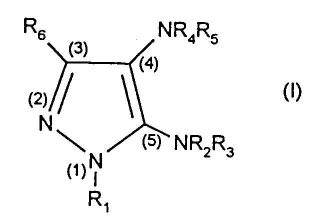 dans laquelle :
- R₁, R₂, R₃, R₄ et R₅, identiques ou différents, représentent un atome d'hydrogène ; un radical alkyle en C₁-C₆ linéaire ou ramifié ; un radical hydroxyalkyle en C₂-C₄ ; un radical aminoalkyle en C₂-C₄ ; un radical phényle ; un radical phényle substitué par un atome d'halogène ou un radical alkyle en C₁-C₄, alcoxy en C₁-C₄, nitro, trifluorométhyle, amino ou alkylamino en C₁-C₄ ; un radical benzyle ; un radical benzyle substitué par un atome d'halogène ou par un radical alkyle en C₁-C₄, alcoxy en C₁-C₄, méthylènedioxy ou amino ; ou un radical 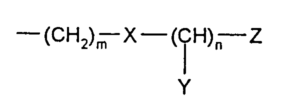 dans lequel m et n sont des nombres entiers, identiques ou différents, compris entre 1 et 3 inclusivement, X représente un atome d'oxygène ou bien le groupement NH, Y représente un atome d'hydrogène ou bien un radical méthyle, et Z représente un radical méthyle, un groupement OR ou NRR' dans lesquels R et R', qui peuvent être identiques ou différent, désignent un atome d'hydrogène, un radical méthyle ou un radical éthyle,
   étant entendu que lorsque R₂ représente un atome d'hydrogène, alors R₃ peut également représenter un radical amino ou alkylamino en C₁-C₄,
- R₆ représente un radical alkyle en C₁-C₆, linéaire ou ramifié ; un radical hydroxyalkyle en C₁-C₄ ; un radical aminoalkyle en C₁-C₄ ; un radical alkyle (C₁-C₄)-aminoalkyle en C₁-C₄ ; un radical dialkyle (C₁-C₄)-aminoalkyle en C₁-C₄ ; un radical hydroxyalkyle (C₁-C₄)-amino alkyle en C₁-C₄ ; un radical alcoxy (C₁-C₄) méthyle ; un radical phényle ; un radical phényle substitué par un atome d'halogène ou par un radical alkyle en C₁-C₄, alcoxy en C₁-C₄, nitro, trifluorométhyle, amino ou alkylamino en C₁-C₄ ; un radical benzyle ; un radical benzyle substitué par un atome d'halogène ou par un radical alkyle en C₁-C₄, alcoxy en C₁-C₄, nitro, trifluorométhyle, amino ou alkylamino en C₁-C₄ ; un hétérocycle choisi parmi le thiophène, le furane et la pyridine, ou encore un radical -(CH₂)ₚ-O-(CH₂)_{q}-OR'', dans lequel p et q sont des nombres entiers, identiques ou différents, compris entre 1 et 3 inclusivement et R'' représente un atome d'hydrogène ou un radical méthyle,
   étant entendu que dans la formule (I) ci-dessus :
- au moins un des radicaux R₂, R₃, R₄ et R₅ représente un atome d'hydrogène,
- lorsque que R₂, respectivement R₄, représente un radical phényle substitué ou non, ou un radical benzyle ou un radical 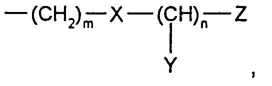 alors R₃, respectivement R₅, ne peut représenter aucun de ces trois radicaux,
- lorsque R₄ et R₅ représentent simultanément un atome d'hydrogène, alors R₁ peut former, avec R₂ et R₃, un hétérocycle hexahydropyrimidinique ou tétrahydroimidazolique éventuellement substitué par un radical alkyle en C₁-C₄ ou 1,2,4-tétrazolique,
- lorsque R₂, R₃, R₄ et R₅ représentent un atome d'hydrogène ou un radical alkyle en C₁-C₆, alors R₁ ou R₆ peut également représenter un reste hétérocyclique 2, 3 ou 4-pyridyle, 2 ou 3-thiényle, 2 ou 3-furyle éventuellement substitué par un radical méthyle ou bien encore un radical cyclohexyle.

Comme indiqué précédemment, les colorations obtenues avec la composition de teinture d'oxydation conforme à l'invention sont puissantes et permettent d'atteindre des nuances allant du rouge au bleu. L'une des caractéristiques essentielles des bases d'oxydation conformes à l'invention, en particulier par rapport à celles décrites dans le document EP-A-375 977 précité, réside dans la présence d'un radical substituant R₆ sur le cycle pyrazole.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Parmi les 4,5-diamino pyrazoles 3-substitués de formule (I), utilisables à titre de base d'oxydation dans les compositions conformes à l'invention, on peut notamment citer :
- le 1-benzyl 4,5-diamino 3-méthyl pyrazole,
- le 4,5-diamino 1-(β-hydroxyéthyl) 3-(4'-méthoxyphényl) pyrazole,
- le 4,5-diamino 1-(β-hydroxyéthyl) 3-(4'-méthylphényl) pyrazole,
- le 4,5-diamino 1-(β-hydroxyéthyl) 3-(3'-méthylphényl) pyrazole,
- le 4,5-diamino 3-méthyl 1-isopropyl pyrazole,
- le 4,5-diamino 3-(4'-méthoxyphényl) 1-isopropyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 3-hydroxyméthyl 1-méthyl pyrazole,
- le 4,5-diamino 1-éthyl 3-hydroxyméthyl pyrazole,
- le 4,5-diamino 3-hydroxyméthyl 1-isopropyl pyrazole,
- le 4,5-diamino 3-hydroxyméthyl 1-ter-butyl pyrazole,
- le 4,5-diamino 3-hydroxyméthyl 1-phényl pyrazole,
- le 4,5-diamino 3-hydroxyméthyl 1-(2'-méthoxyphényl) pyrazole,
- le 4,5-diamino 3-hydroxyméthyl 1-(3'-méthoxyphényl) pyrazole,
- le 4,5-diamino 3-hydroxyméthyl 1-(4'-méthoxyphényl) pyrazole,
- le 1-benzyl 4,5-diamino 3-hydroxyméthyl pyrazole,
- le 4,5-diamino 3-méthyl 1-(2'-méthoxyphényl) pyrazole,
- le 4,5-diamino 3-méthyl 1-(3'-méthoxyphényl) pyrazole,
- le 4,5-diamino 3-méthyl 1-(4'-méthoxyphényl) pyrazole,
- le 3-aminométhyl 4,5-diamino 1-méthyl pyrazole,
- le 3-aminométhyl 4,5-diamino 1-éthyl pyrazole,
- le 3-aminométhyl 4,5-diamino 1-isopropyl pyrazole,
- le 3-aminométhyl 4,5-diamino 1-ter-butyl pyrazole,
- le 4,5-diamino 3-diméthylaminométhyl 1-méthyl pyrazole,
- le 4,5-diamino 3-diméthylaminométhyl 1-éthyl pyrazole,
- le 4,5-diamino 3-diméthylaminométhyl 1-isopropyl pyrazole,
- le 4,5-diamino 3-diméthylaminométhyl 1-ter-butyl pyrazole,
- le 4,5-diamino 3-éthylaminométhyl 1-méthyl pyrazole,
- le 4,5-diamino 3-éthylaminométhyl 1-éthyl pyrazole,
- le 4,5-diamino 3-éthylaminométhyl 1-isopropyl pyrazole,
- le 4,5-diamino 3-éthylaminométhyl 1-ter-butyl pyrazole,
- le 4,5-diamino 3-méthylaminométhyl 1-méthyl pyrazole,
- le 4,5-diamino 3-méthylaminométhyl 1-isopropyl pyrazole,
- le 4,5-diamino 1-éthyl 3-méthylaminométhyl pyrazole,
- le 1-ter-butyl 4,5-diamino 3-méthylaminométhyl pyrazole,
- le 4,5-diamino 3-[(β-hydroxyéthyl)aminométhyl] 1-méthyl pyrazole,
- le 4,5-diamino 3-[(β-hydroxyéthyl)aminométhyl] 1-isopropyl pyrazole,
- le 4,5-diamino 1-éthyl 3-[(β-hydroxyéthyl)aminométhyl] pyrazole,
- le 1-ter-butyl 4,5-diamino 3-[(β-hydroxyéthyl)aminométhyl] pyrazole,
- le 4-amino 5-(β-hydroxyéthyl)amino 1,3-diméthyl pyrazole,
- le 4-amino 5-(β-hydroxyéthyl)amino 1-isopropyl 3-méthyl pyrazole,
- le 4-amino 5-(β-hydroxyéthyl)amino 1-éthyl 3-méthyl pyrazole,
- le 4-amino 5-(β-hydroxyéthyl)amino 1-ter-butyl 3-méthyl pyrazole,
- le 4-amino 5-(β-hydroxyéthyl)amino 1-phényl 3-méthyl pyrazole,
- le 4-amino 5-(β-hydroxyéthyl)amino 1-(2-méthoxyphényl) 3-méthyl pyrazole,
- le 4-amino 5-(β-hydroxyéthyl)amino 1-(3-méthoxyphényl) 3-méthyl pyrazole,
- le 4-amino 5-(β-hydroxyéthyl)amino 1-(4-méthoxyphényl) 3-méthyl pyrazole,
- le 4-amino 5-(β-hydroxyéthyl)amino 1-benzyl 3-méthyl pyrazole,
- le 4-amino 1-éthyl 3-méthyl 5-méthylamino pyrazole,
- le 4-amino 1-ter-butyl 3-méthyl 5-méthylamino pyrazole,
- le 4,5-diamino 1,3-diméthyl pyrazole,
- le 4,5-diamino 3-tert.butyl 1-méthyl pyrazole,
- le 4,5-diamino 1-tert.butyl 3-méthyl pyrazole,
- le 4,5-diamino 1-méthyl 3-phényl pyrazole,
- le 4,5-diamino 1-(β-hydroxyéthyl) 3-méthyl pyrazole,
- le 4,5-diamino 1-(β-hydroxyéthyl) 3-phényl pyrazole,
- le 4,5-diamino 1-méthyl 3-(2'-chlorophényl) pyrazole,
- le 4,5-diamino 1-méthyl 3-(4'-chlorophényl) pyrazole,
- le 4,5-diamino 1-méthyl 3-(3'-trifluorométhylphényl) pyrazole,
- le 4,5-diamino 1,3-diphényl pyrazole,
- le 4,5-diamino 3-méthyl 1-phényl pyrazole,
- le 4-amino 1,3-diméthyl 5-phénylamino pyrazole,
- le 4-amino 1-éthyl 3-méthyl 5-phénylamino pyrazole,
- le 4-amino 1,3-diméthyl 5-méthylamino pyrazole,
- le 4-amino 3-méthyl 1-isopropyl 5-méthylamino pyrazole,
- le 4-amino 3-isobutoxyméthyl 1-méthyl 5-méthylamino pyrazole,
- le 4-amino 3-méthoxyéthoxyméthyl 1-méthyl 5-méthylamino pyrazole,
- le 4-amino 3-hydroxyméthyl 1-méthyl 5-méthylamino pyrazole,
- le 4-amino 1,3-diphényl 5-phénylamino pyrazole,
- le 4-amino 3-méthyl 5-méthylamino 1-phényl pyrazole,
- le 4-amino 1,3-diméthyl 5-hydrazino pyrazole,
- le 5-amino 3-méthyl 4-méthylamino 1-phényl pyrazole,
- le 5-amino 1-méthyl 4-(N,N-méthylphényl)amino 3-(4'-chlorophényl) pyrazole,
- le 5-amino 3-éthyl 1-méthyl 4-(N,N-méthylphényl)amino pyrazole,
- le 5-amino 1-méthyl 4-(N,N-méthylphényl)amino 3-phényl pyrazole,
- le 5-amino 3-éthyl 4-(N,N-méthylphényl)amino pyrazole,
- le 5-amino 4-(N,N-méthylphényl)amino 3-phényl pyrazole,
- le 5-amino 4-(N,N-méthylphényl)amino 3-(4'-méthylphényl) pyrazole,
- le 5-amino 3-(4'-chlorophényl) 4-(N,N-méthylphényl)amino pyrazole,
- le 5-amino 3-(4'-méthoxyphényl) 4-(N,N-méthylphényl)amino pyrazole,
- le 4-amino 5-méthylamino 3-phényl pyrazole,
- le 4-amino 5-éthylamino 3-phényl pyrazole,
- le 4-amino 5-éthylamino 3-(4'-méthylphényl) pyrazole,
- le 4-amino 3-phényl 5-propylamino pyrazole,
- le 4-amino 5-butylamino 3-phényl pyrazole,
- le 4-amino 3-phényl 5-phénylamino pyrazole,
- le 4-amino 5-benzylamino 3-phényl pyrazole,
- le 4-amino 5-(4'-chlorophényl)amino 3-phényl pyrazole,
- le 4-amino 3-(4'-chlorophényl) 5-phénylamino pyrazole,
- le 4-amino 3-(4'-méthoxyphényl) 5-phénylamino pyrazole,
- le 1-(4'-chlorobenzyl) 4,5-diamino 3-méthyl pyrazole,
- le 4,5-diamino 3-hydroxyméthyl 1-isopropyl pyrazole,
- le 4-amino 1-éthyl 3-méthyl 5-méthylamino pyrazole,
- le 4-amino 5-(2'-aminoéthyl)amino 1,3-diméthyl pyrazole,
et leurs sels d'addition avec un acide.

Parmi ces 4,5-diamino pyrazoles 3-substitués, on préfère plus particulièrement :
- le 4,5-diamino 1,3-diméthyl pyrazole,
- le 4,5-diamino 3-méthyl 1-phényl pyrazole,
- le 4,5-diamino 1-méthyl 3-phényl pyrazole,
- le 4-amino 1,3-diméthyl 5-hydrazino pyrazole,
- le 1-benzyl 4,5-diamino 3-méthyl pyrazole,
- le 4,5-diamino 3-tert-butyl 1-méthyl pyrazole,
- le 4,5-diamino 1-tert-butyl 3-méthyl pyrazole,
- le 4,5-diamino 1-(β-hydroxyéthyl) 3-méthyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 1-éthyl 3-hydroxyméthyl pyrazole,
- le 4,5-diamino 3-hydroxyméthyl 1-méthyl pyrazole,
- le 4,5-diamino 3-hydroxyméthyl 1-isopropyl pyrazole,
- le 4,5-diamino 3-méthyl 1-isopropyl pyrazole,
- le 4-amino 5-(2'-aminoéthyl)amino 1,3-diméthyl pyrazole,
et leurs sels d'addition avec un acide.

Le ou les 4,5-diamino pyrazoles 3-substitués de formule (I) ci-dessus représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 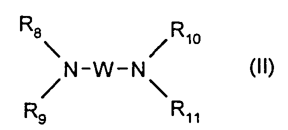 dans laquelle W est un reste propylène éventuellement substitué par un groupement hydroxyle ou un radical alkyle en C₁-C₄ ; R₈, R₉, R₁₀ et R₁₁, identiques ou différents, représentent un atome d'hydrogène, un radical alkyle en C₁-C₄ ou hydroxyalkyle en C₁-C₄.

La composition tinctoriale conforme à l'invention peut encore contenir, en plus des colorants définis ci-dessus, au moins une base d'oxydation additionnelle qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et des bases hétérocycliques différentes des 4,5-diamino pyrazoles 3-substitués utilisés conformément à l'invention.

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR 2 630 438, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols, on peut plus particulièrement citer à titre d'exemple, citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques, les dérivés pyrazoliques différents des 4,5-diamino pyrazoles 3-substitués utilisés conformément à l'invention, et leurs sels d'addition avec un acide.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, soit à l'air, soit à l'aide d'un agent oxydant.

Selon une première forme de mise en oeuvre du procédé de l'invention, la coloration des fibres peut être effectuée sans addition d'un agent oxydant, au seul contact de l'oxygène de l'air. Dans ce cas, la composition tinctoriale peut alors éventuellement contenir des catalyseurs d'oxydation, afin d'accélérer le processus d'oxydation.

A titre de catalyseurs d'oxydation, on peut plus particulièrement citer les sels métalliques tels que les sels de manganèse, de cobalt, de cuivre, de fer, d'argent et de zinc.

De tels composés sont par exemple le diacétate de manganèse tétrahydrate, le dichlorure de manganèse et ses hydrates, le dihydrogénocarbonate de manganèse, l'acétylacétonate de manganèse, le triacétate de manganèse et ses hydrates, le trichlorure de manganèse, le dichlorure de zinc, le diacétate de zinc dihydrate, le carbonate de zinc, le dinitrate de zinc, le sulfate de zinc, le dichlorure de fer, le sulfate de fer, le diacétate de fer, le diacétate de cobalt tétrahydrate, le carbonate de cobalt, le dichlorure de cobalt, le dinitrate de cobalt, le sulfate de cobalt heptahydrate, le chlorure cuivrique, le nitrate d'argent ammoniacal.

Les sels de manganèse sont particulièrement préférés.

Lorsqu'ils sont utilisés, ces sels métalliques sont généralement mis en oeuvre dans des proportions variant entre 0,001 et 4 % en poids d'équivalent métal par rapport au poids total de la composition tinctoriale et de préférence entre 0,005 et 2 % en poids d'équivalent métal par rapport au poids total de la composition tinctoriale.

Selon une deuxième forme de mise en oeuvre du procédé de l'invention, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon cette deuxième forme de mise en oeuvre du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Certains composés de formule (I), utilisés à titre de base d'oxydation dans le cadre de la présente invention, sont nouveaux et, à ce titre, constituent un autre objet de l'invention.

Ces nouveaux 4,5-diamino pyrazoles 3-substitués et leurs sels d'addition avec un acide répondent à la formule (I') suivante : 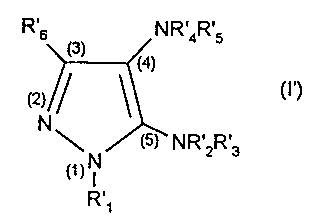 dans laquelle R'₁, R'₂, R'₃, R'₄, R'₅ et R'₆ ont les mêmes significations que celles indiquées précédemment pour les radicaux R₁, R₂, R₃, R₄, R₅ et R₆ dans la formule (I), avec néanmoins les réserves suivantes :
(i) lorsque R'₁ représente un radical méthyle, et que R'₂, R'₄ et R'₅ représentent simultanément un atome d'hydrogène et que R'₃ représente un atome d'hydrogène ou un radical méthyle, alors R'₆ est différent d'un radical hydroxyméthyle, isobutyloxyméthyle, méthoxyéthyloxyméthyle, cyclohexyle, thiophène, pyridine, phényle ou phényle substitué par un radical méthyle ou par un radical trifluorométhyle ou par un atome de chlore ;
(ii) lorsque R'₁ représente un radical phényle non substitué, et que R'₂, R'₃, R'₄ et R'₅ représentent simultanément un atome d'hydrogène, alors R'₆ est différent d'un radical phényle non substitué ;
(iii) lorsque R'₁ représente un radical phényle non substitué, et que R'₆ représente un radical méthyle et que R'₂, R'₄ et R'₅ représentent simultanément un atome d'hydrogène, alors R'₃ est différent d'un atome d'hydrogène, d'un radical méthyle ou phényle non substitué ;
(iv) lorsque R'₁ représente un radical phényle non substitué, et que R'₆ représente un radical méthyle et que R'₄ et R'₅ représentent simultanément un atome d'hydrogène et que R'₂ représente un radical méthyle ou éthyle, alors R'₃ est différent d'un radical phényle non substitué ;
(v) lorsque R'₁ représente un radical phényle non substitué, et que R'₆ représente un radical méthyle et que R'₂, R'₃ et R'₅ représentent simultanément un atome d'hydrogène, alors R'₄ est différent d'un radical méthyle ;
(vi) lorsque R'₂, R'₃, R'₄ et R'₅ représentent simultanément un atome d'hydrogène, et que R'₆ représente un radical méthyle, alors R'₁ est différent d'un radical phényle substitué par un atome de chlore, par un radical trifluoroéthyle, nitro ou pyridyle ;
(vii) lorsque R'₁ représente un atome d'hydrogène, et que R'₆ représente un radical phényle ou un radical phényle substitué par un atome de chlore, par un radical méthyle ou méthoxy et que R'₂, R'₄ et R'₅ représentent simultanément un atome d'hydrogène, alors R'₃ est différent d'un atome d'hydrogène, d'un radical alkyle en C₁-C₄ ou phényle non substitué ;
(viii) lorsque R'₁, R'₂, R'₃, R'₄ et R'₅ représentent simultanément un atome d'hydrogène, alors R'₆ est différent d'un radical méthyle ;
(ix) lorsque R'₁ représente un radical β-hydroxyéthyle, et que R'₂, R'₃, R'₄ et R'₅ représentent simultanément un atome d'hydrogène, alors R'₆ est différent d'un radical méthyle ou phényle non substitué;
(x) lorsque R'₄ représente un radical méthyle, et que R'₅ représente un radical phényle non substitué et que R'₂ et R'₃ représentent simultanément un atome d'hydrogène et que R'₁ représente un atome d'hydrogène ou un radical méthyle, alors R'₆ est différent d'un radical phényle non substitué ou d'un radical phényle substitué par un radical méthyle, éthyle, méthoxy ou par un atome de chlore ;
(xi) lorsque R'₁ représente un radical ter-butyle, et que R'₂, R'₃, R'₄ et R'₅ représentent simultanément un atome d'hydrogène, alors R'₆ est différent d'un radical méthyle ;
(xii) lorsque R'₁ représente un radical pyridyle, et que R'₂, R'₄ et R'₅ représentent simultanément un atome d'hydrogène et que R'₃ représente un atome d'hydrogène ou un radical méthyle, alors R'₆ est différent d'un radical méthyle ou phényle non substitué ;
(xiii) lorsque R'₁ représente un radical méthyle, éthyle ou 4-aminophényle, et que R'₂, R'₄ et R'₅ représentent simultanément un atome d'hydrogène et que R'₃ représente un atome d'hydrogène ou un radical phényle non substitué alors R'₆ est différent d'un radical méthyle ;
(xiv) lorsque R'₁ représente un radical isopropyle, et que R'₂, R'₄ et R'₅ représentent simultanément un atome d'hydrogène, alors au moins un des radicaux R'₃ et R'₆ est différent d'un radical méthyle ;
(xv) lorsque R'₁ représente un atome d'hydrogène ou un radical phényle non substitué, et que R'₂, R'₄ et R'₅ représentent simultanément un atome d'hydrogène et que R'₃ représente un radical benzyle ou un radical phényle substitué par un radical méthyle ou par un atome de chlore, alors R'₆ est différent d'un radical méthyle ou phényle non substitué.

Parmi les nouveaux composés de formule (I'), on peut notamment citer :
- le 1-benzyl 4,5-diamino 3-méthyl pyrazole,
- le 4,5-diamino 1-(β-hydroxyéthyl) 3-(4'-méthoxyphényl) pyrazole,
- le 4,5-diamino 1-(β-hydroxyéthyl) 3-(4'-méthylphényl) pyrazole,
- le 4,5-diamino 1-(β-hydroxyéthyl) 3-(3'-méthylphényl) pyrazole,
- le 4,5-diamino 3-méthyl 1-isopropyl pyrazole,
- le 4,5-diamino 3-(4'-méthoxyphényl) 1-isopropyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 3-hydroxyméthyl 1-méthyl pyrazole,
- le 4,5-diamino1-éthyl 3-hydroxyméthyl pyrazole,
- le 4,5-diamino 3-hydroxyméthyl 1-isopropyl pyrazole,
- le 4,5-diamino 3-hydroxyméthyl 1-ter-butyl pyrazole,
- le 4,5-diamino 3-hydroxyméthyl 1-phényl pyrazole,
- le 4,5-diamino 3-hydroxyméthyl 1-(2'-méthoxyphényl) pyrazole,
- le 4,5-diamino 3-hydroxyméthyl 1-(3'-méthoxyphényl) pyrazole,
- le 4,5-diamino 3-hydroxyméthyl 1-(4'-méthoxyphényl) pyrazole,
- le 1-benzyl 4,5-diamino 3-hydroxyméthyl pyrazole,
- le 4,5-diamino 3-méthyl 1-(2'-méthoxyphényl) pyrazole,
- le 4,5-diamino 3-méthyl 1-(3'-méthoxyphényl) pyrazole,
- le 4,5-diamino 3-méthyl 1-(4'-méthoxyphényl) pyrazole,
- le 3-aminométhyl 4,5-diamino 1-méthyl pyrazole,
- le 3-aminométhyl 4,5-diamino 1-éthyl pyrazole,
- le 3-aminométhyl 4,5-diamino 1-isopropyl pyrazole,
- le 3-aminométhyl 4,5-diamino 1-ter-butyl pyrazole,
- le 4,5-diamino 3-diméthylaminométhyl 1-méthyl pyrazole,
- le 4,5-diamino 3-diméthylaminométhyl 1-éthyl pyrazole,
- le 4,5-diamino 3-diméthylaminométhyl 1-isopropyl pyrazole,
- le 4,5-diamino 3-diméthylaminométhyl 1-ter-butyl pyrazole,
- le 4,5-diamino 3-éthylaminométhyl 1-méthyl pyrazole,
- le 4,5-diamino 3-éthylaminométhyl 1-éthyl pyrazole,
- le 4,5-diamino 3-éthylaminométhyl 1-isopropyl pyrazole,
- le 4,5-diamino 3-éthylaminométhyl 1-ter-butyl pyrazole,
- le 4,5-diamino 3-méthylaminométhyl 1-méthyl pyrazole,
- le 4,5-diamino 3-méthylaminométhyl 1-isopropyl pyrazole,
- le 4,5-diamino 1-éthyl 3-méthylaminométhyl pyrazole,
- le 1-ter-butyl 4,5-diamino 3-méthylaminométhyl pyrazole,
- le 4,5-diamino 3-[(β-hydroxyéthyl)aminométhyl] 1-méthyl pyrazole,
- le 4,5-diamino 3-[(β-hydroxyéthyl)aminométhyl] 1-isopropyl pyrazole,
- le 4,5-diamino 1-éthyl 3-[(β-hydroxyéthyl)aminométhyl] pyrazole,
- le 1-ter-butyl 4,5-diamino 3-[(β-hydroxyéthyl)aminométhyl] pyrazole,
- le 4-amino 5-(β-hydroxyéthyl)amino 1,3-diméthyl pyrazole,
- le 4-amino 5-(β-hydroxyéthyl)amino 1-isopropyl 3-méthyl pyrazole,
- le 4-amino 5-(β-hydroxyéthyl)amino 1-éthyl 3-méthyl pyrazole,
- le 4-amino 5-(β-hydroxyéthyl)amino 1-ter-butyl 3-méthyl pyrazole,
- le 4-amino 5-(β-hydroxyéthyl)amino 1-phényl 3-méthyl pyrazole,
- le 4-amino 5-(β-hydroxyéthyl)amino 1-(2'-méthoxyphényl) 3-méthyl pyrazole,
- le 4-amino 5-(β-hydroxyéthyl)amino 1-(3'-méthoxyphényl) 3-méthyl pyrazole,
- le 4-amino 5-(β-hydroxyéthyl)amino 1-(4'-méthoxyphényl) 3-méthyl pyrazole,
- le 4-amino 5-(β-hydroxyéthyl)amino 1-benzyl 3-méthyl pyrazole,
- le 4-amino 1-éthyl 3-méthyl 5-méthylamino pyrazole,
- le 4-amino 1-ter-butyl 3-méthyl 5-méthylamino pyrazole,
- le 1-(4'-chlorobenzyl) 4,5-diamino 3-méthyl pyrazole,
- le 4,5-diamino 3-hydroxyméthyl 1-isopropyl pyrazole,
- le 4-amino 1-éthyl 3-méthyl 5-méthylamino pyrazole,
- le 4-amino 5-(2'-aminoéthyl)amino 1,3-diméthyl pyrazole,
et leurs sels d'addition avec un acide.

Parmi les nouveaux composés de formule (I'), on préfère plus particulièrement :
- le 1-benzyl 4,5-diamino 3-méthyl pyrazole,
- le 4,5-diamino 1-éthyl 3-méthyl pyrazole,
- le 4,5-diamino 1-éthyl 3-(4'-méthoxyphényl) pyrazole
- le 4,5-diamino 1-éthyl 3-hydroxyméthyl pyrazole,
- le 4,5-diamino 3-hydroxyméthyl 1-méthyl pyrazole,
- le 4,5-diamino 3-hydroxyméthyl 1-isopropyl pyrazole,
- le 4,5-diamino 3-méthyl 1-isopropyl pyrazole,
- le 4-amino 5-(2'-aminoéthyl)amino 1,3-diméthyl pyrazole,
et leurs sels d'addition avec un acide.

L'invention a également pour objet les procédés de préparation des composés nouveaux de formule (I').

Lorsque R'₆ représente un radical méthyle et que R'₁ est différent d'un atome d'hydrogène, (composés de formule (I'A) ci-dessous), on utilise de préférence le procédé A répondant au schéma de synthèse suivant : 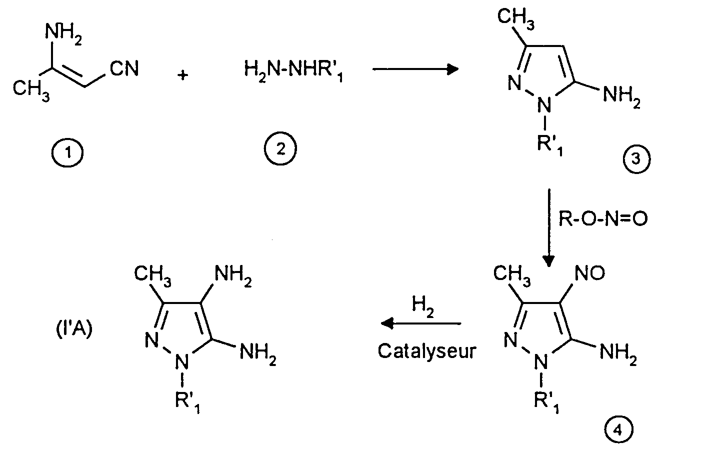 consistant à faire réagir, dans une première étape, un 3-aminocrotononitrile ① avec une hydrazine monosubstituée ②, à une température généralement supérieure à 90°C, et de préférence comprise entre 95 et 150°C, dans un solvant alcoolique, puis, dans une deuxième étape, à nitroser le 5-aminopyrazole ③ en position 4, par réaction avec un nitrite minéral ou organique pour donner le 5-amino 4-nitrosopyrazole ④ qui conduit, dans une troisième étape, par hydrogénation catalytique aux 4,5-diamino pyrazoles de formule (I'A).

Pour un bon contrôle de la température lors de la première étape, on préfère généralement opérer au reflux du solvant utilisé. Parmi les alcools utilisés à titre de solvant réactionnel, on peut plus particulièrement citer le n-propanol, le 1-butanol, le 2-butanol, le 2-méthyl 1-butanol, le 3-méthyl 1-butanol, le 2-méthyl 1-propanol, le n-pentanol, le 2-pentanol, le 3-méthyl 3-pentanol, le 4-méthyl 2-pentanol ou encore le 2-éthyl 1-butanol.

Parmi les nitrites minéraux, on peut par exemple utiliser le nitrite de sodium ou de potassium, en milieu acide acétique aqueux, à une température comprise de préférence entre 0 et 5°C.

Parmi les nitrites organiques, on peut par exemple utiliser le nitrite d'isoamyle, en conduisant la réaction à température ambiante, dans un alcool inférieur en présence d'un acide tel que l'acide chlorhydrique ou l'acide acétique.

L'hydrogénation catalytique des composés ④ est de préférence effectuée dans un alcool inférieur, en présence d'un catalyseur tel que le palladium sur charbon, à un température généralement comprise entre 20 et 100°C.

Lorsque R'₆ est différent d'un radical méthyle et lorsque R'₁ est différent d'un atome d'hydrogène, (composés de formule (I'B) ci-dessous), on utilise de préférence le procédé B répondant au schéma de synthèse suivant : 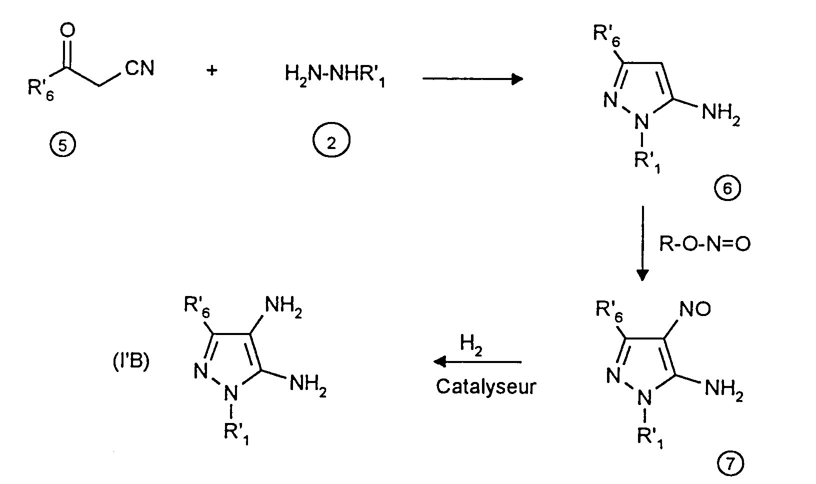 consistant à faire réagir, dans une première étape, un β-cétoacétonitrile ⑤ avec une hydrazine monosubstituée ②, à une température généralement comprise entre 20 et 150°C, dans un solvant alcoolique, pour obtenir le 5-aminopyrazole ⑥ qui est ensuite nitrosé en position 4, dans une deuxième étape, pour donner un 4-nitro 5-amino pyrazole ⑦ qui est ensuite lui-même hydrogéné, dans une troisième étape, pour conduire aux 4,5-diamino pyrazoles de formule (I'B).

Les solvants utilisés selon ce procédé B sont les mêmes que ceux cités pour le procédé A décrit précédemment.

Les réactions de nitrosation et d'hydrogénation sont réalisées selon les conditions décrites pour le procédé A décrit précédemment.

Lorsque R'₆ représente un radical à fort encombrement stérique, (composés de formule (I'C) ci-dessous), on utilise de préférence le procédé C répondant au schéma de synthèse suivant : 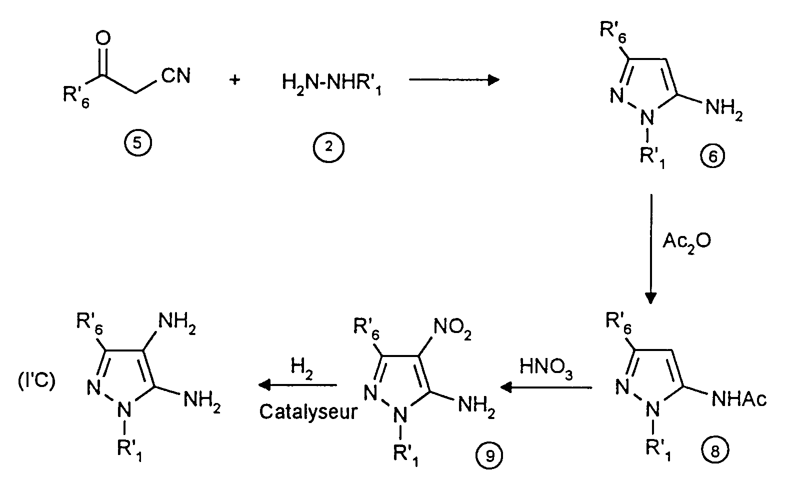 consistant à faire réagir, dans une première étape, un β-cétoacétonitrile ⑤ avec une hydrazine monosubstituée ② pour obtenir un 5-aminopyrazole ⑥ selon les conditions opératoires mentionnées pour le procédé B décrit précédemment. Le 5-aminopyrazole ⑥ est ensuite acétylé en position 5, dans une deuxième étape, pour conduire à un 5-acétylamino pyrazole ⑧ qui est lui-même ensuite nitré en position 4 et désacétylé en position 5, dans une troisième étape, pour donner un 5-amino 4-nitro pyrazole ⑨, de préférence par l'acide nitrique fumant en milieu sulfurique concentré, à une température de préférence comprise entre 0 et 5°C. Le 5-amino 4-nitro pyrazole ⑨ est ensuite hydrogéné, dans une quatrième étape, selon les conditions opératoires mentionnées dans le procédé A ci-dessus, pour conduire aux 4,5-diamino pyrazoles de formule (I'C).

Lorsque l'un des radicaux R'₂ ou R'₃ est différent d'un atome d'hydrogène, (composés de formule (I'D) ci-dessous), on utilise de préférence le procédé D répondant au schéma de synthèse suivant : 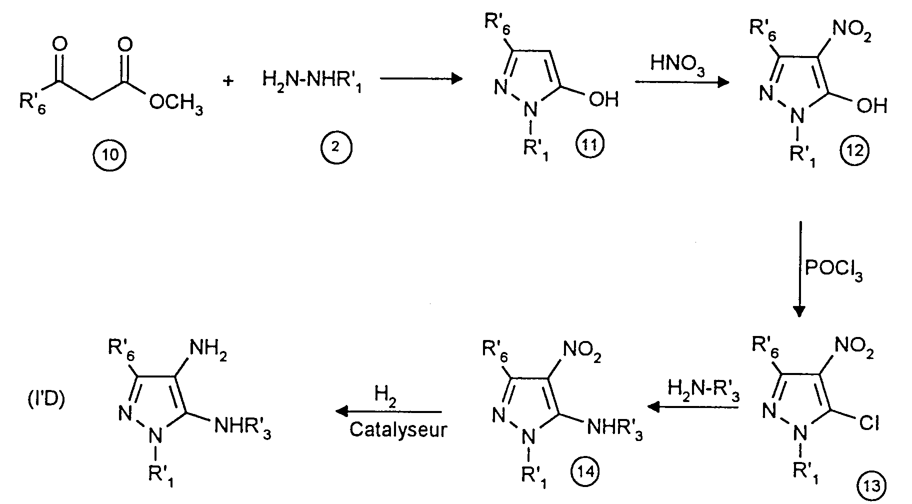 consistant à faire réagir, dans une première étape, un β-cétoester (10) avec une hydrazine ②, pour obtenir un 5-hydroxypyrazole (11), qui est en équilibre avec sa forme tautomère pyrazol-5-one, tel que décrit par exemple dans Org. Synth., Edward C. TAYLOR, (1976), 55, 73-7. Le 5-hydroxypyrazole (11) est ensuite nitré en position 4, dans une deuxième étape, puis chloré en position 5, dans une troisième étape, selon la méthode telle que décrite par exemple dans le brevet US 4 025 530. Le 5-chloro 4-nitro pyrazole (13) conduit ensuite, dans une quatrième étape, en présence d'une amine primaire H₂N-R'₃, à un 5-amino 4-nitro pyrazole (14), puis dans une cinquième étape, par hydrogénation catalytique, selon la méthode décrite ci-dessus pour le procédé A, aux 4,5-diamino pyrazoles de formule (I'D).

Les exemples qui suivent sont destinés à illustrer l'invention sans pour autant en limiter la portée.

### EXEMPLES DE PREPARATION

### EXEMPLE DE PREPARATION 1 : Synthèse du dichlorhydrate du 4,5-diamino 1,3-diméthyl pyrazole

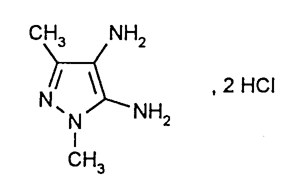

### a) Préparation du 5-amino 1,3-diméthyl pyrazole

A une solution de 16,5 g (0,2 mole) de 3-aminocrotononitrile dans 40 cm³ de n-pentanol, on a ajouté 12,9 g (0,28 mole) de méthylhydrazine. La solution a été portée au reflux pendant 3 heures. Le pentanol, ainsi que l'excès de méthylhydrazine, ont ensuite été distillés sous pression réduite. Le précipité beige obtenu a été repris par 150 cm³ d'heptane, essoré sur verre fritté puis séché sous vide à une température de 40°C. On a obtenu 13,5 g de 5-amino 1,3-diméthyl pyrazole sous forme d'un solide beige dont le point de fusion était compris entre 80 et 81°C.

### b) Préparation du 5-amino 1,3-diméthyl 4-nitroso pyrazole

A une solution de 11,1 g (0,1 mole) de 5-amino 1,3-diméthyl pyrazole, obtenu à l'étape précédente, dans 125 cm³ d'éthanol absolu, on a ajouté goutte à goutte 1 cm³ d'acide chlorhydrique 12 N, puis 13,5 cm³ de nitrite d'isoamyle à 0°C. La solution a ensuite été ramenée et laissée à température ambiante pendant 4 heures. Le milieu réactionnel a alors été filtré sur verre fritté et le précipité obtenu a été lavé par 100 cm³ d'éther isopropylique. Après séchage sous vide à température ambiante, on a obtenu 7,5 g de 5-amino 1,3-diméthyl 4-nitroso pyrazole sous forme d'un solide orangé dont le point de fusion était compris entre 169 et 171°C.

### c) Préparation du dichlorhydrate du 4,5-diamino 1,3-diméthyl pyrazole

A une solution de 7 g (0,05 mole) de 5-amino 1,3-diméthyl pyrazole, obtenu à l'étape précédente, dans 200 cm3 d'éthanol, on a ajouté 1,2 g de palladium sur charbon à 5 % en poids et contenant 50 % d'eau. La suspension a été placée dans un hydrogénateur sous une pression d'hydrogène de 10 bars, à une température de 75°C, pendant 3 heures, sous vive agitation. Le milieu réactionnel a été versé dans une solution de 50 cm³ d'éthanol et de 17 cm³ d'acide chlorhydrique 12 N refroidie à 0°C. Cette solution a ensuite été clarifiée par filtration sur verre fritté, puis évaporée à sec sous pression réduite. Le solide brun obtenu a été repris au reflux de 45 cm³ d'éthanol chlorhydrique 5 N et de 13 cm³ d'eau, puis on a refroidi à température ambiante. Le précipité blanc obtenu a été essoré sur verre fritté, puis séché sous vide à température ambiante. On a obtenu 5,7 g de dichlorhydrate de 4,5-diamino 1,3-diméthyl pyrazole, sous forme de cristaux blancs dont le point de décomposition était compris entre 210 et 212 °C. L'analyse élémentaire calculée pour C₅H₁₀N₄, 2 HCl était :

| % | C | H | N | Cl |
|---|---|---|---|---|
| Calculé | 30,17 | 6,08 | 28,14 | 35,62 |
| Trouvé | 30,15 | 6,08 | 28,07 | 35,77 |

### EXEMPLE DE PREPARATION 2 : Synthèse du dichlorhydrate du 4,5-diamino 3-méthyl 1-phényl pyrazole

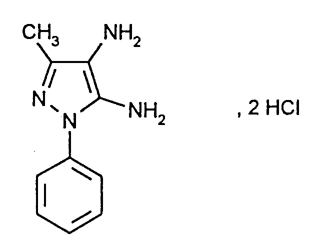

### a) Préparation du 5-amino 3-méthyl 4-nitroso 1-phényl pyrazole

A une solution de 17,3 g (0,1 mole) de 5-amino 3-méthyl 1-phényl pyrazole dans 200 cm³ d'éthanol absolu, on a ajouté goutte à goutte 0,5 cm³ d'acide chlorhydrique 12 N puis 13,5 cm³ de nitrite d'isoamyle à 0°C. La solution a ensuite été ramenée et laissée à température ambiante pendant 4 heures. Un solide orange a cristallisé. Ce solide a été essoré sur verre fritté et lavé par 100 cm³ d'éther isopropylique. Après séchage sous vide à température ambiante, on a obtenu 17 g de 5-amino 3-méthyl 4-nitroso 1-phényl pyrazole sous forme d'un solide orange dont le point de fusion était compris entre 202 et 204 °C.

### b) Préparation du dichlorhydrate de 4,5-diamino 3-méthyl 1-phényl pyrazole

A une solution de 10 g (0,05 mole) de 5-amino 3-méthyl 4-nitroso 1-phényl pyrazole, obtenu à l'étape précédente, dans 80 cm³ d'éthanol absolu et 20 cm³ d'acide chlorhydrique 2 N, on a ajouté 1,2 g de palladium sur charbon à 5% en poids contenant 50 % d'eau. La suspension a été placée dans un hydrogénateur, sous une pression d'hydrogène de 10 bars, à une température de 75°C, sous vive agitation, pendant 3 heures. Le contenu de l'hydrogénateur a ensuite été récupéré et filtré sur verre fritté. Le filtrat a été coulé dans une solution à 0°C de 70 cm³ d'éthanol absolu et de 30 cm³ d'acide chlorhydrique 12 N puis évaporé à sec. Le résidu a été repris par 100 cm³ d'éther isopropylique : un solide beige a cristallisé. Ce solide a été essoré sur verre fritté puis lavé par 100 cm³ d'éther isopropylique et purifié par recristallisation dans un mélange de 100 cm³ d'eau et de 50 cm³ d'une solution éthanolique d'acide chlorhydrique 3,5 M. Le solide cristallisé a été essoré sur verre fritté, lavé par 100 cm³ d'éther isopropylique et séché sous vide à température ambiante. On a obtenu 8 g de dichlorhydrate de 4,5-diamino 3-méthyl 1-phényl pyrazole sous forme d'un solide blanc, dont le point de fusion était compris entre 208 et 210 °C. L'analyse élémentaire calculée pour C₁₀H₁₂N₄, 2 HCl était :

| % | C | H | N | Cl |
|---|---|---|---|---|
| Calculé | 45,99 | 5,40 | 21,45 | 27,15 |
| Trouvé | 46,17 | 5,40 | 21,32 | 27,10 |

### EXEMPLE DE PREPARATION 3 : Synthèse du dichlorhydrate du 4,5-diamino 1-méthyl 3-phényl pyrazole

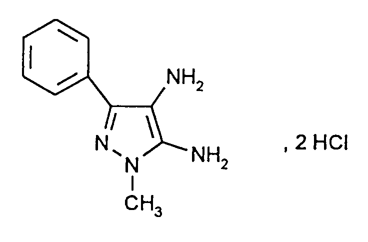

### a) Préparation du 5-amino 1-méthyl 3-phényl pyrazole

A une solution de 29 g (0,2 mole) de benzoylacétonitrile dans 100 cm³ de n-pentanol, on a ajouté 14,7 cm³ (0,28 mole) de méthylhydrazine et on a chauffé au reflux pendant 2 heures. On a ensuite distillé le n-pentanol ainsi que l'excès de méthylhydrazine sous pression réduite. On a obtenu un solide beige qui a été repris par 100 cm³ d'heptane à température ambiante et essoré sur verre fritté. Après séchage sous vide à une température de 40 °C, on a obtenu 27 g de 5-amino 1-méthyl 3-phényl pyrazole sous forme d'un solide beige dont le point de fusion était compris entre 104 et 106 °C.

### b) Préparation du 5-amino 1-méthyl 4-nitroso 3-phényl pyrazole

A une solution de 17,3 g (0,1 mole) de 5-amino 1-méthyl 3-phényl pyrazole, obtenu à l'étape précédente, dans 200 cm³ d'éthanol absolu, on a ajouté goutte à goutte 0,5 cm³ d'acide chlorhydrique 12 N puis 13,5 cm³ de nitrite d'isoamyle à 0°C. La solution a ensuite été ramenée et laissée à température ambiante pendant 4 heures. Un solide orange a cristaliisé. Il a été essoré sur verre fritté et lavé par 100 cm³ d'éther isopropylique. Après séchage sous vide à température ambiante, on a obtenu 17 g de 5-amino 1-méthyl 4-nitroso 3-phényl pyrazole sous forme d'un solide orange dont le point de fusion était compris entre 224 et 226°C.

### c) Préparation du dichlorhydrate du 4,5-diamino 1-méthyl 3-phényl pyrazole

A une solution de 10 g (0,05 mole) de 5-amino 1-méthyl 4-nitroso 3-phényl pyrazole, obtenu à l'étape précédente, dans 80 cm³ d'éthanol absolu et 20 cm3 d'une solution d'acide chlorhydrique 2 N, on a ajouté 1,2 g de palladium sur charbon à 5 % en poids contenant 50 % d'eau. La suspension a été placée dans un hydrogénateur, sous une pression d'hydrogène de 10 bars, à une température de 75 °C, pendant 3 heures sous vive agitation. Le contenu de l'hydrogénateur a été récupéré et filtré sur verre fritté. Le filtrat a été coulé dans une solution de 70 cm³ d'éthanol absolu et de 30 cm³ d'acide chlorhydrique 12 N refroidie à 0°C. Un solide blanc a précipité. Ce précipité a été essoré sur verre fritté puis lavé par 100 cm³ d'éther isopropylique. Après séchage sous vide à température ambiante, on a obtenu 10 g de dichlorhydrate de 4,5-diamino 1-méthyl 3-phényl pyrazole qui ont été purifiés par recristallisation dans un mélange de 30 cm³ d'eau et de 50 cm³ d'une solution éthanolique d'acide chlorhydrique 3,5 M. Le solide recristallisé a été essoré sur verre fritté, lavé par 100 cm³ d'éther isopropylique et séché sous vide à température ambiante. On a obtenu 8 g de dichlorhydrate de 4,5-diamino 1-méthyl 3-phényl pyrazole, sous la forme d'un solide blanc, dont le point de fusion était compris entre 218 et 220 °C, l'analyse élémentaire calculée pour C₁₀H₁₂N₄, 2 HCl était :

| % | C | H | N | Cl |
|---|---|---|---|---|
| Calculé | 45,99 | 5,40 | 21,45 | 27,15 |
| Trouvé | 46,27 | 5,60 | 21,32 | 27,10 |

### EXEMPLE DE PREPARATION 4 : Synthèse du dichlorhydrate du 4-amine 1,3-diméthyl 5-hydrazino pyrazole

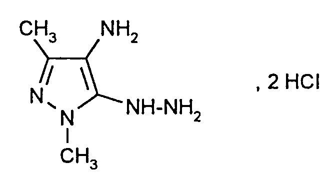

Une suspension de 8,6 g (0,05 mole) de 1,3-diméthyl 5-hydrazino 4-nitro pyrazole et de 1,5 g de palladium sur charbon à 5 % en poids contenant 50 % d'eau dans 200 cm³ d'éthanol a été placée dans un hydrogénateur. Après agitation sous une pression d'hydrogène de 10 bars, à une température de 75°C pendant 3 heures, le milieu réactionnel a été versé dans une solution de 60 cm³ d'éthanol et de 20 cm³ d'acide chlorhydrique 12 N refroidie à 0°C. La solution a été clarifiée par filtration sur verre fritté, puis évaporée à sec sous pression réduite. Le solide brun obtenu a été repris au reflux de 50 cm³ d 'éthanol chlorhydrique 5 N et de 14 cm³ d'eau, puis on a refroidi à température ambiante. Le précipité blanc obtenu a été essoré sur verre fritté, puis séché sous vide à température ambiante. On a obtenu 9,5 g de dichlorhydrate de 4-amino 1,3-diméthyl 5-hydrazino pyrazole sous forme de cristaux blancs dont le point de fusion était compris entre 202 et 204°C. L'analyse élémentaire calculée pour C₅H₁₁N₅, 2 HCl était :

| % | C | H | N | Cl |
|---|---|---|---|---|
| Calculé | 28,05 | 6,12 | 32,71 | 33,12 |
| Trouvé | 28,52 | 6,20 | 32,96 | 32,88 |

### EXEMPLE DE PREPARATION 5 : Synthèse du dichlorhydrate du 1-benzyl 4,5-diamino 3-méthyl pyrazole

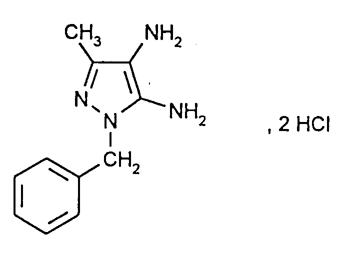

### a) Préparation du 5-amino 1-benzyl 3-méthyl pyrazole

A une solution de 16,4 g (0,2 mole) de 3-amino crotononitrile dans 100 cm³ de n-pentanol, on a ajouté 26,9 g (0,22 mole) de benzylhydrazine puis on a chauffé au reflux pendant 12 heures. Le n-pentanol a ensuite été distillé sous pression réduite, on a obtenue une huile épaisse qui a été purifiée par chromatographie sur gel de silice. On a obtenu un solide jaune pâle qui a cristallisé dans l'éther isopropylique et qui a été essoré sur verre fritté. Après séchage sous vide à 40°C, on a obtenu 17 g du produit attendu sous forme d'un solide jaune pâle dont le point de fusion était compris entre 76 et 78°C.

### b) Préparation du 5-amino 1-benzyl 3-méthyl 4-nitroso pyrazole

A une solution de 18,7 g (0,1 mole) de 5-amino 1-benzyl 3-méthyl pyrazole, obtenu à l'étape précédente, dans 200 cm³ d'éthanol absolu, on a ajouté goutte à goutte 0,5 cm³ d'acide chlorhydrique 12 N, puis 13,5 cm³ de nitrite d'isoamyle à 0°C. La solution a ensuite été ramenée et laissée à température ambiante pendant 4 heures. Un solide orange a cristallisé. Il a été essoré sur verre fritté et lavé par 100 cm³ d'éthanol puis par 100 cm³ d'éther isopropylique. Après séchage sous vide à température ambiante, on a obtenu 13 g du produit attendu sous la forme d'un solide orange dont le point de fusion était compris entre 178 et 180°C.

### c) Préparation du dichlorhydrate du 1-benzyl 4,5-diamino 3-méthyl pyrazole

A une solution de 5 g (0,02 mole) de 5-amino 1-benzyl 3-méthyl 5-nitroso pyrazole, obtenu à l'étape précédente, dans 200 cm³ de méthanol, on a ajouté 0,9 g de palladium sur charbon à 5 % en poids et contenant 50 % d'humidité. La suspension a été placée dans un hydrogénateur, sous une pression de 20 bars d'hydrogène, à température ambiante, pendant 3 heures, sous vive agitation. Le contenu de l'hydrogénateur a été prélevé et filtré sur verre fritté. Le filtrat a ensuite été coulé dans 100 cm³ d'une solution d'éthanol chlorhydrique 3,5 M. Cette solution a été concentrée sous vide. On a obtenu une huile épaisse, qui a cristallisé par ajout de 50 cm³ d'acétone. On a obtenu un solide qui a été essoré sur verre fritté.
Après séchage sous vide à température ambiante, on a obtenu 6 g de dichlorhydrate du 1-benzyl 4,5-diamino 3-méthyl pyrazole sous forme d'un solide blanc dont le point de fusion était compris entre 190 et 192°C. L'analyse élémentaire calculée pour C₁₁H₁₄N₄, 2 HCl était :

| % | C | H | N | Cl |
|---|---|---|---|---|
| Calculé | 48,01 | 5,86 | 20,36 | 25,77 |
| Trouvé | 48,03 | 5,90 | 20,40 | 25,75 |

### EXEMPLE DE PREPARATION 6 : Synthèse du dichlorhydrate du 4,5-diamino 1-méthyl 3-ter-butyl pyrazole

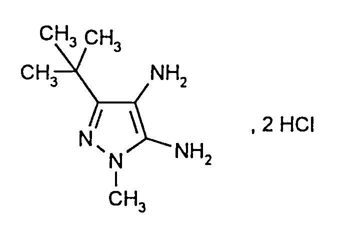

### a) Préparation du 5-amino 1-méthyl 3-ter-butyl pyrazole

A une solution de 12,5 g (0,1 mole) de 4,4-diméthyl 3-oxo pentanitrile dans 50 cm³ de n-propanol, on a ajouté, à température ambiante, 4,6 g (0,1 mole) de méthylhydrazine. La milieu réactionnel a été porté au reflux pendant 1 heure, puis refroidi à température ambiante. Le solide blanc obtenu a été essoré sur verre fritté puis lavé à l'éther isopropylique. Après séchage sous vide à 40°C, on a obtenu 10 g du produit attendu sous forme d'un solide beige dont le point de fusion était de 157°C.

### b) Préparation du 5-acétamido 1-méthyl 3-ter-butyl pyrazole

A une solution de 10 g (0,065 mole) de 5-amino 1-méthyl 3-ter-butyl pyrazole, obtenu à l'étape précédente, dans 25 cm³ d'acide acétique, on a ajouté, à température ambiante, 13,5 cm³ (0,13 mole) d'anhydride acétique. Après 1 heure d'agitation, le milieu réactionnel a été versé sur 100 cm³ de glace. On a extrait la solution par trois fois 100 cm³ de dichlorométhane, séché la phase organique sur sulfate de sodium, puis distillé celle-ci sous vide à l'évaporateur rotatif. Le solide obtenu a été repris par 100 cm³ d'éther isopropylique, essoré sur verre fritté, puis séché sous vide à 40°C. On a obtenu 12 g du produit attendu sous forme d'un solide beige que l'on a recristallisé dans 30 cm³ d'acétate d'éthyle pour isoler 8,5 g du produit attendu sous forme de cristaux blancs dont le point de fusion était de 138°C.

### c) Préparation du 5-amino 1-méthyl 4-nitro 3-ter-butyl pyrazole

A 30 cm³ d'acide sulfurique concentré, on a ajouté 8,5 g (0,044 mole) de 5-acétamido 1-méthyl 3-ter-butyl pyrazole à 5°C sous vive agitation, puis 2,5 cm³ (0,066 mole) d'acide nitrique fumant. Après 2 heures d'agitation, le mélange réactionnel a été versé sur 100 g de glace et agité pendant 30 minutes. Le solide obtenu a été essoré sur verre fritté, lavé par 20 cm³ d'eau, puis séché sous vide à 40°C. On a obtenu 8,5 g du produit attendu sous forme d'un solide jaune dont le point de fusion était de 124°C.

### d) Préparation du dichlorhydrate du 4,5-diamino 1-méthyl 3-ter-butyl pyrazole

Une suspension de 8,5 g (0,035 mole) de 5-amino 1-méthyl 4-nitro 3-ter-butyl pyrazole et de 1,5 g de palladium sur charbon à 5% en poids et contenant 50 % d'eau dans 200 cm³ d'éthanol a été placée dans un hydrogénateur. Après agitation pendant 3 heures, sous une pression d'hydrogène de 10 bars, à une température de 75°C, le milieu réactionnel a été versé dans une solution, préalablement refroidie à 0°C, de 60 cm3 d'éthanol et de 20 cm³ d'acide chlorhydrique 12 N. La solution a été clarifiée par filtration sur verre fritté, puis évaporée à sec sous pression réduite. Le solide brun obtenu a été repris au reflux de 35 cm³ d'éthanol chlorhydrique 5 N et de 11 cm³ d'eau, puis refroidi à température ambiante. Les cristaux blancs obtenus ont été essorés sur verre fritté puis séchés sous vide à température ambiante. On a obtenu 4,9 g du produit attendu sous forme de cristaux blancs dont le point de fusion était de 260°C. L'analyse élémentaire pour C₈H₁₆N₄, 2 HCl était :

| % | C | H | N | Cl |
|---|---|---|---|---|
| Calculé | 39,84 | 7,52 | 23,23 | 29,40 |
| Trouvé | 39,73 | 7,63 | 23,16 | 29,20 |

### EXEMPLE DE PREPARATION 7 : Synthèse du dichlorhydrate du 4,5-diamino 3-méthyl 1-ter-butyl pyrazole

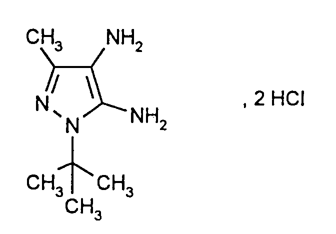

### a) Préparation du 5-amino 3-méthyl 1-ter-butyl pyrazole

A une solution de 16,4 g (0,2 mole) de 3-aminocrotononitrile dans 100 cm³ de n-pentanol, on a ajouté 19,4 g (0,22 mole) de ter-butylhydrazine. Cette solution a été chauffée au reflux pendant 20 heures. Le n-pentanol a ensuite été distillé sous pression réduite. On a obtenu un solide jaune pâle qui a été repris par 100 cm³ d'éther isopropylique à température ambiante et essoré sur verre fritté. Après séchage sous vide à 40°C, on a obtenu 18 g du produit attendu sous forme d'un solide jaune pâle dont le point de fusion était compris entre 172 et 175°C.

### b) Préparation du 5-amino 3-méthyl 4-nitroso 1-ter-butyl pyrazole

A une solution de 15,3 g (0,1 mole) de 5-amino 3-méthyl 1-ter-butyl pyrazole, obtenu à l'étape précédente, dans 200 cm3 d'éthanol absolu, on a ajouté goutte à goutte 0,5 cm³ d'acide chlorhydrique 12 N, puis 13,5 cm³ (0,1 mole) de nitrite d'isoamyle à 0°C. La solution a ensuite été ramenée et laissée à température ambiante pendant 4 heures.
L'éthanol a été évaporé sous pression de 175 m·bars, à 40°C. Un solide orange a cristallisé dans l'heptane à 0°C, puis a été essoré sur verre fritté. Après séchage sous vide à température ambiante, on a^{o}obtenu 11 g du produit attendu, sous forme d'un solide orange dont le point de fusion était de 120°C.

### c) Préparation du dichlorhydrate du 4,5-diamino 3-méthyl 1-ter-butyl pyrazole

A une solution de 9 g (0,05 mole) de 5-amino 3-méthyl 4-nitroso 1-ter-butyl pyrazole, obtenu à l'étape précédente, dans 600 cm³ d'éthanol absolu, on a ajouté 2 g de palladium sur charbon à 5 % en poids et contenant 50 % d'eau. La suspension a été placée dans un hydrogénateur, sous une pression de 20 bars d'hydrogène, à température ambiante, pendant 4 heures, sous vive agitation. Le contenu de l'hydrogénateur a été prélevé et filtré sur verre fritté. Le filtrat a ensuite été coulé dans 100 cm³ d'une solution d'éthanol chlorhydrique 3,5 M. Cette solution a été concentrée sous vive jusqu'à début de cristallisation. Les cristaux ont ensuit été lavés avec une solution d'éthanol chlorhydrique 3,5 M, puis essoré sur verre fritté. On a obtenu un solide blanc qui a été recristallisé dans un mélange de 40 cm³ d'éthanol chlorhydrique 3,5 M et de 12 cm³ d'eau distillée. Après séchage sous vide à température ambiante, on a obtenu 8 g du produit attendu sous forme de cristaux blancs dont le point de fusion était compris entre 252 et 255°C. L'analyse élémentaire pour C₈H₁₆N₄, 2 HCl était :

| % | C | H | N | Cl |
|---|---|---|---|---|
| Calculé | 39,84 | 7,52 | 23,23 | 29,40 |
| Trouvé | 39,70 | 7,49 | 23,37 | 29,44 |

### EXEMPLE DE PREPARATION 8 : Synthèse du dichlorhydrate du 4,5-diamino 1-β-hydroxyéthyl 3-méthyl pyrazole

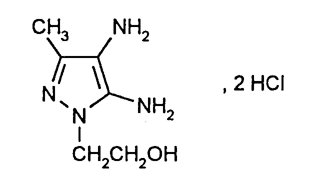

### a) Préparation du 5-amino 1-(β-hydroxyéthyl) 3-méthyl pyrazole

A une solution de 16,4 g (0,2 mole) de 3-aminocrotononitrile dans 100 cm³ de n-pentanol, on a ajouté 16,7 g (0,22 mole) de β-hydroxyéthylhydrazine, puis on a chauffé au reflux pendant 12 heures. Le n-pentanol a ensuite été distillé sous pression réduite. On a obtenu une huile épaisse qui cristallise par ajout de 150 cm³ d'éther isopropylique. On a obtenu un solide beige qui a été essoré sur verre fritté. Après séchage sous vide à 40°C, on a obtenu 18 g du produit attendu sous forme d'un solide beige dont le point de fusion était compris entre 66 et 68°C.

### b) Préparation du 5-amino 1-(β-hdroxéthyl)3-méthyl 4-nitroso pyrazole

A une solution de 14,1 g (0,1 mole) de 5-amino 1-(β-hydroxyéthyl) 3-méthyl pyrazole, obtenu à l'étape précédente, dans 200 cm³ d'éthanol absolu, on a ajouté goutte à goutte 0,5 cm³ d'acide chlorhydrique 12 N, puis 13,5 cm³ de nitrite d'isoamyle à 0°C. La solution a ensuite été ramenée et laissée à température ambiante pendant 4 heures. Un solide rouge a cristallisé et a été essoré sur verre fritté puis lavé par 100 cm³ d'éthanol puis par 100 cm³ d'éther isopropylique. Après séchage sous vide à température ambiante, on a obtenu 10,5 g du produit attendu sous la forme de cristaux rouges dont le point de fusion était compris entre 170 et 175°C.

### c) Préparation du dichlorhydrate du 4,5-diamino 1-(β-hydroxyéthyl) 3-méthyl pyrazole

A une solution de 8,5 g (0,05 mole) du produit obtenu à l'étape précédente, dans 800 cm³ de méthanol, on a ajouté 2 g de palladium sur charbon à 5 % en poids et contenant 50 % d'humidité. La suspension a été placée dans un hydrogénateur, sous une pression de 20 bars d'hydrogène, à 30°C, pendant 4 heures sous vive agitation.
Le contenu de l'hydrogénateur a ensuite été prélevé et filtré sur verre fritté dans 100 cm³ d'une solution d'éthanol chlorhydrique 6 M. Cette solution a été concentrée sous vide. On a obtenu une huile épaisse qui a cristallisé par ajout de 50 cm³ d'éther isopropylique. On a obtenu un solide blanc qui a été recristallisé dans un mélange de 45 cm³ d'éthanol chlorhydrique 6 M et 3,5 cm³ d'eau distillée.
Après séchage sous vide à température ambiante, on a obtenu 7,5 g du produit attendu, sous forme de cristaux blanc dont le point de fusion était compris entre 190 et 193°C. L'analyse élémentaire calculée pour C₆H₁₂N₄O, 2 HCl était :

| % | C | H | N | O | Cl |
|---|---|---|---|---|---|
| Calculé | 31,46 | 6,16 | 24,45 | 6,98 | 30,95 |
| Trouvé | 31,44 | 6,21 | 24,10 | 7,07 | 30,98 |

### EXEMPLE DE PREPARATION 9 : Synthèse du dichlorhydrate du 4-amine 5-(2'-aminoéthyl)amino 1,3-diméthyl pyrazole

### a) Préparation du chlorhydrate du 5-[(2'-aminoéthyl)amino] 1,3-diméthyl 4-nitro pyrazole

A une solution de 17,5 g (0,1 mole) de 5-chloro 1,3-diméthyl pyrazole dans 200 cm³ de n-propanol, on a ajouté 7,3 cm³ (0,11 mole) d'éthylènediamine. Cette solution a été chauffée au reflux pendant 4 heures. La solution a été ensuite ramenée et laissée à température ambiante pendant 15 heures. Un solide jaune vif a cristallisé, puis a été essoré sur verre fritté. Après séchage sous vide à 40°C, on a obtenu 19 g du produit attendu, sous forme d'un solide jaune dont le point de fusion était 235°C.

### b) Préparation du dichlorhydrate du 4-amino 5-(2'-aminoéthyl)amino 1,3-diméthyl pyrazole

A une solution de 10 g (0,04 mole) de chlorhydrate du 5-[(2'-aminoéthyl)amino] 1,3-diméthyl 4-nitro pyrazole, obtenu à l'étape précédente, dans 20 cm³ de méthanol, on a ajouté 2 g de palladium sur charbon à 5 % en poids et contenant 50 % d'eau. La suspension a été placée dans un hydrogénateur sous une pression de 10 bars d'hydrogène à 40°C pendant 3 heures sous vive agitation. Le contenu de l'hydrogénateur a été prélevé et filtré sur verre fritté dans 100 cm³ d'une solution d'éthanol chlorhydrique 3,5 M. Cette solution a été concentrée sous vide. On a obtenu une huile épaisse qui a cristallisée par ajout de 50 cm³ d'éther isopropylique. Le solide formé a été essoré sur verre fritté puis lavé par 20 cm³ d'éther isopropylique et purifié par recristallisation dans un mélange de 33 cm³ d'éthanol absolu et de 16 cm³ d'acide chlorhydrique 6 M. Le solide cristallisé a été essoré sur verre fritté lavé par 50 cm³ d'éther isopropylique et séché sous vide à température ambiante. On a obtenu 5 g du dichlorhydrate du 4-amino 5-(2'-aminoéthyl)amino 1,3-diméthyl pyrazole sous forme de solide blanc dont le point de fusion était compris entre 238 et 240°C.

L'analyse élémentaire pour C₇H₁₅N₅, 2 HCl était :

| **%** | **C** | **H** | **N** | **C1** |
|---|---|---|---|---|
| **Calculé** | 34,72 | 7,08 | 28,92 | 29,28 |
| **Trouvé** | 34,70 | 7,05 | 28,89 | 29,50 |

### EXEMPLES D'APPLICATION

### EXEMPLES 10 A 18 DE TEINTURE EN MILIEU ALCALIN

On a préparé les compositions tinctoriales, conformes à l'invention, suivantes (teneurs en grammes) :

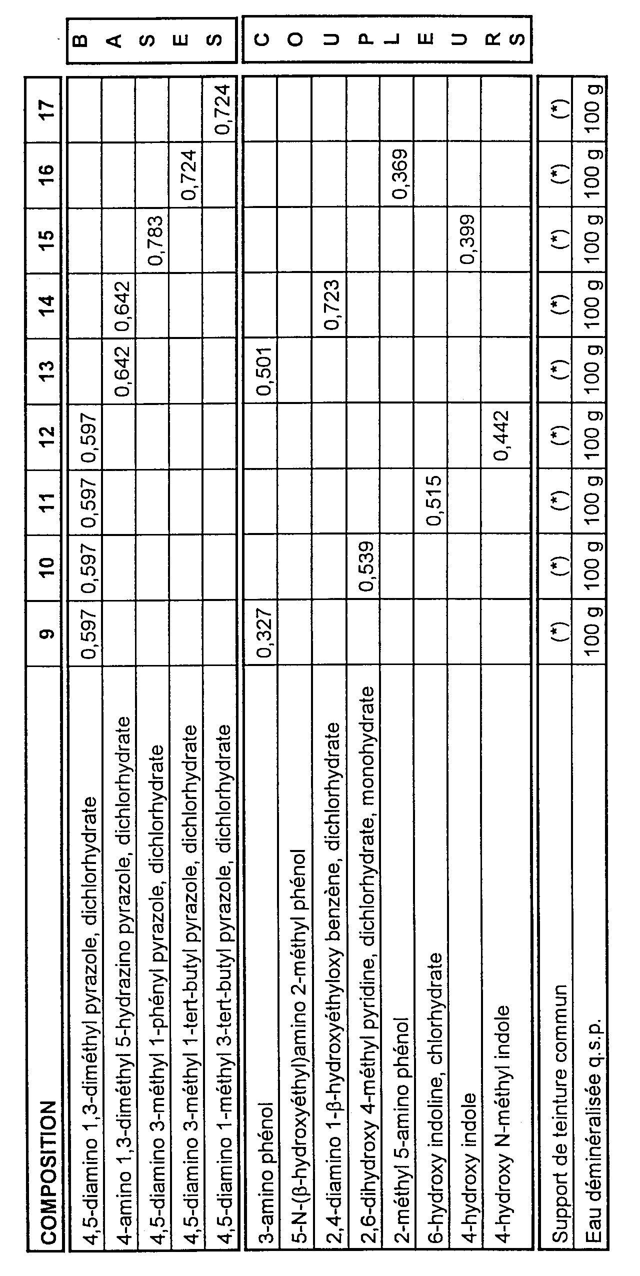

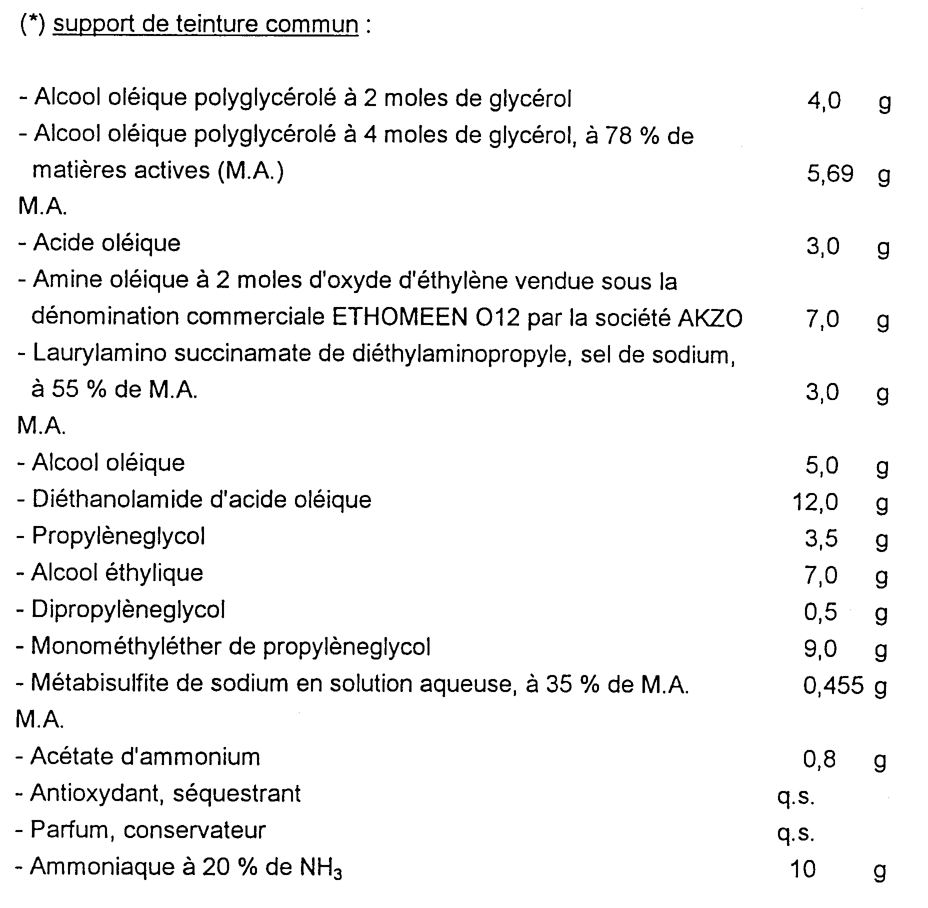

Au moment de l'emploi, on a mélangé chaque composition tinctoriale 9 à 17 avec une quantité égale en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes dans les nuances figurant dans le tableau ci-dessous:

| **EXEMPLE** | **pH DU MELANGE** | **NUANCE SUR CHEVEUX NATURELS** |
|---|---|---|
| **10** | 9,8 | Irisé |
| **11** | 9,9 | Doré cuivré |
| **12** | 9,9 | Blond légèrement irisé |
| **13** | 9,9 | Violet |
| **14** | 10,0 | Irisé |
| **15** | 9,8 | Bleu violacé |
| **16** | 9,8 | Violine |
| **17** | 9,7 | Irisé |
| **18** | 9,9 | Bleu |

### EXEMPLES 19 ET 20 DE TEINTURE EN MILIEU ACIDE

On a préparé les compositions tinctoriales, conformes à l'invention, suivantes (teneurs en grammes) :

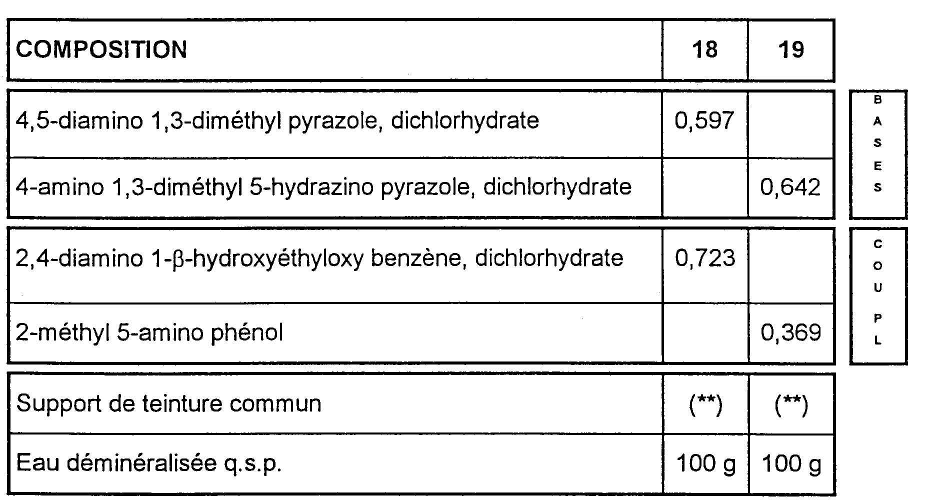

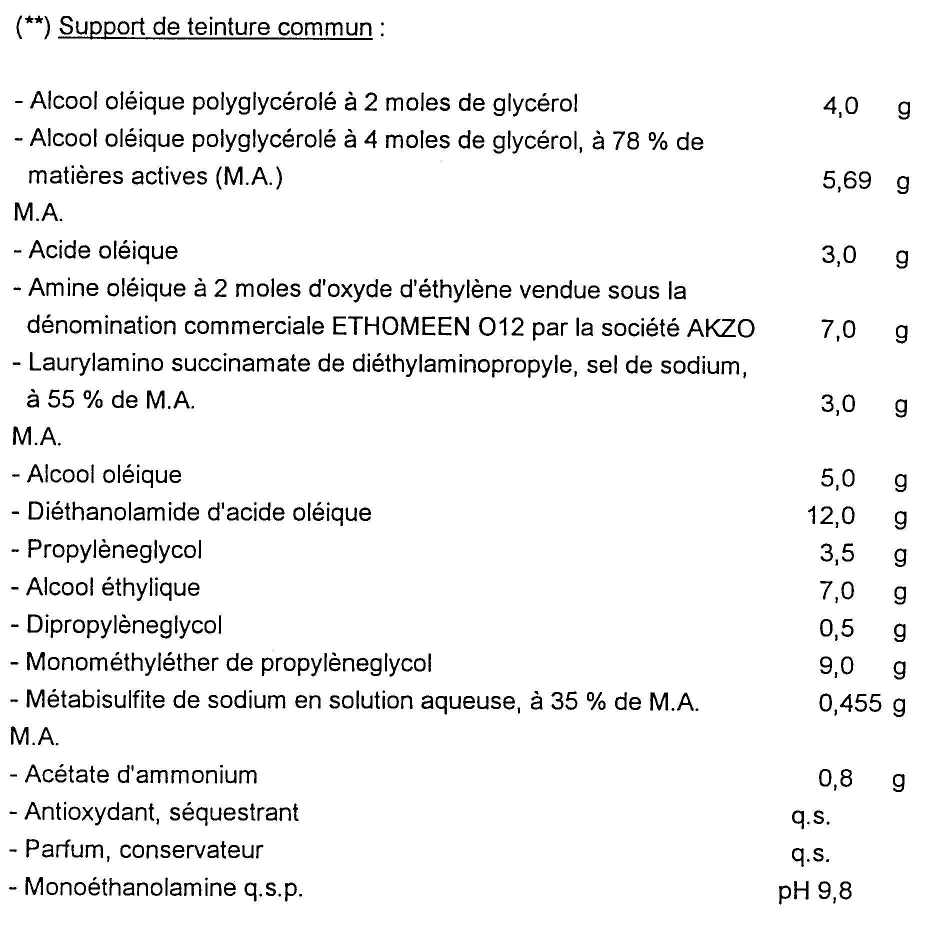

Au moment de l'emploi, on a mélangé chaque composition tinctoriale avec une quantité égale en poids d'une composition oxydante constituée par une solution d'eau oxygénée à 20 volumes (6 % en poids), et dont le pH avait été ajusté entre 1 et 1,5 par 2,5 g d'acide orthophosphorique pour 100 g d'eau oxygénée.

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

Les mèches de cheveux ont été teintes dans les nuances figurant dans le tableau ci-dessous :

| **EXEMPLE** | **pH DU MELANGE** | **NUANCE SUR CHEVEUX NATURELS** |
|---|---|---|
| **19** | 6,9 | Bleu violacé |
| **20** | 6,8 | Irisé |

### EXEMPLE 21 DE TEINTURE A L'AIR

On a préparé la composition tinctoriale, conforme à l'invention, suivante :

| | |
|---|---|
| - Dichlorhydrate de 4,5-diamino 1,3-diméthyl pyrazole | 0,398 g |
| - Dichlorhydrate de 2,4-diamino 1-β-hydroxyéthyloxy benzène | 0,482 g |
| - Alcool éthylique | 10 g |
| - Monoéthanolamine | 2 g |
| - Eau déminéralisée q.s.p. | 100 g |

Cette composition a été appliquée pendant 30 minutes sur des mèches de cheveux gris naturels à 90 % de blancs. Après rinçage et séchage, les mèches ont été teintes dans une nuance cendrée violacée.

## Revendications

1. Composition pour la teinture d'oxydation des fibres kératiniques et en particulier des fibres kératiniques humaines telles que les cheveux, caractérisée par le fait qu'elle comprend, dans un milieu approprié pour la teinture, au moins un 4,5-diamino pyrazole 3-substitué de formule (I) suivante à titre de base d'oxydation et/ou au moins un de ses sels d'addition avec un acide : 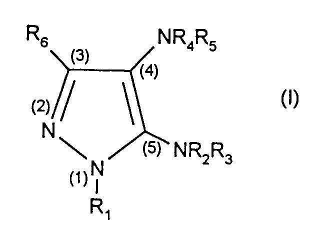 dans laquelle :
- R₁, R₂, R₃, R₄ et R₅, identiques ou différents, représentent un atome d'hydrogène ; un radical alkyle en C₁-C₆ linéaire ou ramifié ; un radical hydroxyalkyle en C₂-C₄ ; un radical aminoalkyle en C₂-C₄ ; un radical phényle ; un radical phényle substitué par un atome d'halogène ou un radical alkyle en C₁-C₄, alcoxy en C₁-C₄, nitro, trifluorométhyle, amino ou alkylamino en C₁-C₄ ; un radical benzyle ; un radical benzyle substitué par un atome d'halogène ou par un radical alkyle en C₁-C₄, alcoxy en C₁-C₄, méthylènedioxy ou amino ; ou un radical 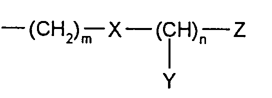 dans lequel m et n sont des nombres entiers, identiques ou différents, compris entre 1 et 3 inclusivement, X représente un atome d'oxygène ou bien le groupement NH, Y représente un atome d'hydrogène ou bien un radical méthyle, et Z représente un radical méthyle, un groupement OR ou NRR' dans lesquels R et R', qui peuvent être identiques ou différent, désignent un atome d'hydrogène, un radical méthyle ou un radical éthyle,
étant entendu que lorsque R₂ représente un atome d'hydrogène, alors R₃ peut également représenter un radical amino ou alkylamino en C₁-C₄,
- R₆ représente un radical alkyle en C₁-C₆, linéaire ou ramifié ; un radical hydroxyalkyle en C₁-C₄ ; un radical aminoalkyle en C₁-C₄ ; un radical alkyle (C₁-C₄)-aminoalkyle en C₁-C₄ ; un radical dialkyle (C₁-C₄)-aminoalkyle en C₁-C₄ ; un radical hydroxyalkyle (C₁-C₄)-amino alkyle en C₁-C₄ ; un radical alcoxy (C₁-C₄) méthyle ; un radical phényle ; un radical phényle substitué par un atome d'halogène ou par un radical alkyle en C₁-C₄, alcoxy en C₁-C₄, nitro, trifluorométhyle, amino ou alkylamino en C₁-C₄ ; un radical benzyle ; un radical benzyle substitué par un atome d'halogène ou par un radical alkyle en C₁-C₄, alcoxy en C₁-C₄, nitro, trifluorométhyle, amino ou alkylamino en C₁-C₄ ; un hétérocycle choisi parmi le thiophène, le furane et la pyridine, ou encore un radical -(CH₂)ₚ-O-(CH₂)_{q}-OR'', dans lequel p et q sont des nombres entiers, identiques ou différents, compris entre 1 et 3 inclusivement et R'' représente un atome d'hydrogène ou un radical méthyle,
étant entendu que dans la formule (I) ci-dessus :
- au moins un des radicaux R₂, R₃, R₄ et R₅ représente un atome d'hydrogène,
- lorsque que R₂, respectivement R₄, représente un radical phényle substitué ou non, ou un radical benzyle ou un radical 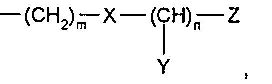 alors R₃, respectivement R₅, ne peut représenter aucun de ces trois radicaux,
- lorsque R₄ et R₅ représentent simultanément un atome d'hydrogène, alors R₁ peut former, avec R₂ et R₃, un hétérocycle hexahydropyrimidinique ou tétrahydroimidazolique éventuellement substitué par un radical alkyle en C₁-C₄ ou 1,2,4-tétrazolique,
- lorsque R₂, R₃, R₄ et R₅ représentent un atome d'hydrogène ou un radical alkyle en C₁-C₆, alors R₁ ou R₆ peut également représenter un reste hétérocyclique 2, 3 ou 4-pyridyle, 2 ou 3-thiényle, 2 ou 3-furyle éventuellement substitué par un radical méthyle ou bien encore un radical cyclohexyle.

2. Composition selon la revendication 1, caractérisée par le fait que les 4,5-diamino pyrazoles 3-substitués de formule (I) sont choisis parmi :
- le 1-benzyl 4,5-diamino 3-méthyl pyrazole,
- le 4,5-diamino 1-(β-hydroxyéthyl) 3-(4'-méthoxyphényl) pyrazole,
- le 4,5-diamino 1-(β-hydroxyéthyl) 3-(4'-méthylphényl) pyrazole,
- le 4,5-diamino 1-(β-hydroxyéthyl) 3-(3'-méthylphényl) pyrazole,
- le 4,5-diamino 3-méthyl 1-isopropyl pyrazole,
- le 4,5-diamino 3-(4'-méthoxyphényl) 1-isopropyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 3-hydroxyméthyl 1-méthyl pyrazole,
- le 4,5-diamino1-éthyl 3-hydroxyméthyl pyrazole,
- le 4,5-diamino 3-hydroxyméthyl 1-isopropyl pyrazole,
- le 4,5-diamino 3-hydroxyméthyl 1-ter-butyl pyrazole,
- le 4,5-diamino 3-hydroxyméthyl 1-phényl pyrazole,
- le 4,5-diamino 3-hydroxyméthyl 1-(2'-méthoxyphényl) pyrazole,
- le 4,5-diamino 3-hydroxyméthyl 1-(3'-méthoxyphényl) pyrazole,
- le 4,5-diamino 3-hydroxyméthyl 1-(4'-méthoxyphényl) pyrazole,
- le 1-benzyl 4,5-diamino 3-hydroxyméthyl pyrazole,
- le 4,5-diamino 3-méthyl 1-(2'-méthoxyphényl) pyrazole,
- le 4,5-diamino 3-méthyl 1-(3'-méthoxyphényl) pyrazole,
- le 4,5-diamino 3-méthyl 1-(4'-méthoxyphényl) pyrazole,
- le 3-aminométhyl 4,5-diamino 1-méthyl pyrazole,
- le 3-aminométhyl 4,5-diamino 1-éthyl pyrazole,
- le 3-aminométhyl 4,5-diamino 1-isopropyl pyrazole,
- le 3-aminométhyl 4,5-diamino 1-ter-butyl pyrazole,
- le 4,5-diamino 3-diméthylaminométhyl 1-méthyl pyrazole,
- le 4,5-diamino 3-diméthylaminométhyl 1-éthyl pyrazole,
- le 4,5-diamino 3-diméthylaminométhyl 1-isopropyl pyrazole,
- le 4,5-diamino 3-diméthylaminométhyl 1-ter-butyl pyrazole,
- le 4,5-diamino 3-éthylaminométhyl 1-méthyl pyrazole,
- le 4,5-diamino 3-éthylaminométhyl 1-éthyl pyrazole,
- le 4,5-diamino 3-éthylaminométhyl 1-isopropyl pyrazole,
- le 4,5-diamino 3-éthylaminométhyl 1-ter-butyl pyrazole,
- le 4,5-diamino 3-méthylaminométhyl 1-méthyl pyrazole,
- le 4,5-diamino 3-méthylaminométhyl 1-isopropyl pyrazole,
- le 4,5-diamino 1-éthyl 3-méthylaminométhyl pyrazole,
- le 1-ter-butyl 4,5-diamino 3-méthylaminométhyl pyrazole,
- le 4,5-diamino 3-[(β-hydroxyéthyl)aminométhyl] 1-méthyl pyrazole,
- le 4,5-diamino 3-[(β-hydroxyéthyl)aminométhyl] 1-isopropyl pyrazole,
- le 4,5-diamino 1-éthyl 3-[(β-hydroxyéthyl)aminométhyl] pyrazole,
- le 1-ter-butyl 4,5-diamino 3-[(β-hydroxyéthyl)aminométhyl] pyrazole,
- le 4-amino 5-(β-hydroxyéthyl)amino 1,3-diméthyl pyrazole,
- le 4-amino 5-(β-hydroxyéthyl)amino 1-isopropyl 3-méthyl pyrazole,
- le 4-amino 5-(β-hydroxyéthyl)amino 1-éthyl 3-méthyl pyrazole,
- le 4-amino 5-(β-hydroxyéthyl)amino 1-ter-butyl 3-méthyl pyrazole,
- le 4-amino 5-(β-hydroxyéthyl)amino 1-phényl 3-méthyl pyrazole,
- le 4-amino 5-(β-hydroxyéthyl)amino 1-(2-méthoxyphényl) 3-méthyl pyrazole,
- le 4-amino 5-(β-hydroxyéthyl)amino 1-(3-méthoxyphényl) 3-méthyl pyrazole,
- le 4-amino 5-(β-hydroxyéthyl)amino 1-(4-méthoxyphényl) 3-méthyl pyrazole,
- le 4-amino 5-(β-hydroxyéthyl)amino 1-benzyl 3-méthyl pyrazole,
- le 4-amino 1-éthyl 3-méthyl 5-méthylamino pyrazole,
- le 4-amino 1-ter-butyl 3-méthyl 5-méthylamino pyrazole,
- le 4,5-diamino 1,3-diméthyl pyrazole,
- le 4,5-diamino 3-tert-butyl 1-méthyl pyrazole,
- le 4,5-diamino 1-tert-butyl 3-méthyl pyrazole,
- le 4,5-diamino 1-méthyl 3-phényl pyrazole,
- le 4,5-diamino 1-(β-hydroxyéthyl) 3-méthyl pyrazole,
- le 4,5-diamino 1-(β-hydroxyéthyl) 3-phényl pyrazole,
- le 4,5-diamino 1-méthyl 3-(2'-chlorophényl) pyrazole,
- le 4,5-diamino 1-méthyl 3-(4'-chlorophényl) pyrazole,
- le 4,5-diamino 1-méthyl 3-(3'-trifluorométhylphényl) pyrazole,
- le 4,5-diamino 1,3-diphényl pyrazole,
- le 4,5-diamino 3-méthyl 1-phényl pyrazole,
- le 4-amino 1,3-diméthyl 5-phénylamino pyrazole,
- le 4-amino 1-éthyl 3-méthyl 5-phénylamino pyrazole,
- le 4-amino 1,3-diméthyl 5-méthylamino pyrazole,
- le 4-amino 3-méthyl 1-isopropyl 5-méthylamino pyrazole,
- le 4-amino 3-isobutoxyméthyl 1-méthyl 5-méthylamino pyrazole,
- le 4-amino 3-méthoxyéthoxyméthyl 1-méthyl 5-méthylamino pyrazole,
- le 4-amino 3-hydroxyméthyl 1-méthyl 5-méthylamino pyrazole,
- le 4-amino 1,3-diphényl 5-phénylamino pyrazole,
- le 4-amino 3-méthyl 5-méthylamino 1-phényl pyrazole,
- le 4-amino 1,3-diméthyl 5-hydrazino pyrazole,
- le 5-amino 3-méthyl 4-méthylamino 1-phényl pyrazole,
- le 5-amino 1-méthyl 4-(N,N-méthylphényl)amino 3-(4'-chlorophényl) pyrazole,
- le 5-amino 3-éthyl 1-méthyl 4-(N,N-méthylphényl)amino pyrazole,
- le 5-amino 1-méthyl 4-(N,N-méthylphényl)amino 3-phényl pyrazole,
- le 5-amino 3-éthyl 4-(N,N-méthylphényl)amino pyrazole,
- le 5-amino 4-(N,N-méthylphényl)amino 3-phényl pyrazole,
- le 5-amino 4-(N,N-méthylphényl)amino 3-(4'-méthylphényl) pyrazole,
- le 5-amino 3-(4'-chlorophényl) 4-(N,N-méthylphényl)amino pyrazole,
- le 5-amino 3-(4'-méthoxyphényl) 4-(N,N-méthylphényl)amino pyrazole,
- le 4-amino 5-méthylamino 3-phényl pyrazole,
- le 4-amino 5-éthylamino 3-phényl pyrazole,
- le 4-amino 5-éthylamino 3-(4'-méthylphényl) pyrazole,
- le 4-amino 3-phényl 5-propylamino pyrazole,
- le 4-amino 5-butylamino 3-phényl pyrazole,
- le 4-amino 3-phényl 5-phénylamino pyrazole,
- le 4-amino 5-benzylamino 3-phényl pyrazole, - le 4-amino 5-(4'-chlorophényl)amino 3-phényl pyrazole,
- le 4-amino 3-(4'-chlorophényl) 5-phénylamino pyrazole,
- le 4-amino 3-(4'-méthoxyphényl) 5-phénylamino pyrazole,
- le 1-(4'-chlorobenzyl) 4,5-diamino 3-méthyl pyrazole,
- le 4,5-diamino 3-hydroxyméthyl 1-isopropyl pyrazole,
- le 4-amino 1-éthyl 3-méthyl 5-méthylamino pyrazole,
- le 4-amino 5-(2'-aminoéthyl)amino 1,3-diméthyl pyrazole,
et leurs sels d'addition avec un acide.

3. Composition selon la revendication 2, caractérisée par le fait que les 4,5-diamino pyrazoles 3-substitués de formule (I) sont choisis parmi :
- le 4,5-diamino 1,3-diméthyl pyrazole,
- le 4,5-diamino 3-méthyl 1-phényl pyrazole,
- le 4,5-diamino 1-méthyl 3-phényl pyrazole,
- le 4-amino 1,3-diméthyl 5-hydrazino pyrazole,
- le 1-benzyl 4,5-diamino 3-méthyl pyrazole,
- le 4,5-diamino 3-tert-butyl 1-méthyl pyrazole,
- le 4,5-diamino 1-tert-butyl 3-méthyl pyrazole,
- le 4,5-diamino 1-(β-hydroxyéthyl) 3-méthyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 1-éthyl 3-hydroxyméthyl pyrazole,
- le 4,5-diamino 3-hydroxyméthyl 1-méthyl pyrazole,
- le 4,5-diamino 3-hydroxyméthyl 1-isopropyl pyrazole,
- le 4,5-diamino 3-méthyl 1-isopropyl pyrazole,
- le 4-amino 5-(2'-aminoéthyl)amino 1,3-diméthyl pyrazole,
et leurs sels d'addition avec un acide.

4. Composition selon l'une quelconque des revendications précédentes, caractérisée par le fait que le ou les 4,5-diamino pyrazoles 3-substitués de formule (I) représentent de 0,0005 à 12 % en poids du poids total de la composition tinctoriale.

5. Composition selon la revendication 4, caractérisée par le fait que le ou les 4,5-diamino pyrazoles 3-substitués de formule (I) représentent de 0,005 à 6 % en poids du poids total de la composition tinctoriale.

6. Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

7. Composition selon l'une quelconque des revendications précédentes, caractérisée par le fait qu'elle présente un pH compris entre 3 et 12.

8. Composition selon l'une quelconque des revendications précédentes, caractérisée par le fait qu'elle renferme au moins une base d'oxydation additionnelle choisie parmi les paraphénylènediamines, les bis-phénylalkylènediamines, les para-aminophénols, les ortho-aminophénols et des bases hétérocycliques différentes des 4,5-diamino pyrazoles 3-substitués de formule (I).

9. Composition selon la revendication 8, caractérisée par le fait que la ou les bases d'oxydation additionnelles représentent de 0,0005 à 12 % en poids du poids total de la composition tinctoriale.

10. Composition selon l'une quelconque des revendications précédentes, caractérisée par le fait qu'elle renferme au moins un coupleur et/ou au moins un colorant direct.

11. Composition selon la revendication 10, caractérisée par le fait que le ou les coupleurs sont choisis parmi les métaphénylènediamines, les méta-aminophénols, les métadiphénols et les coupleurs hétérocycliques, et leurs sels d'addition avec un acide.

12. Composition selon la revendication 10 ou 11, caractérisée par le fait que le ou les coupleurs représentent de 0,0001 à 10% en poids du poids total de la composition tinctoriale.

13. Composition selon l'une quelconque des revendications précédentes, caractérisée par le fait que les sels d'addition avec un acide sont choisis parmi les chlorhydrates, les bromhydrates, les sulfates et les tartrates, les lactates et les acétates.

14. Procédé de teinture des fibres kératiniques et en particulier des fibres kératiniques humaines telles que les cheveux caractérisé par le fait que l'on applique sur ces fibres au moins une composition tinctoriale telle que définie à l'une quelconque des revendications 1 à 13, pendant un temps suffisant pour développer la coloration désirée, soit à l'air, soit à l'aide d'un agent oxydant.

15. Procédé selon la revendication 14, caractérisé par le fait que la coloration est révélée au seul contact de l'oxygène de l'air.

16. Procédé selon la revendication 15, caractérisé par le fait que la coloration est révélée au seul contact de l'oxygène de l'air, en présence de catalyseurs d'oxydation.

17. Procédé selon la revendication 16, caractérisé par le fait que les catalyseurs d'oxydation sont des sels métalliques.

18. Procédé selon la revendication 14, caractérisé par le fait que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

19. Procédé selon la revendication 18, caractérisé par le fait que l'agent oxydant est choisi parmi le peroxyde d'hydrogène, le peroxyde d'urée, les bromates de métaux alcalins, les persels tels que les perborates et persulfates.

20. Dispositif à plusieurs compartiments, ou "kit" de teinture à plusieurs compartiments, dont un premier compartiment renferme une composition tinctoriale telle que définie à l'une quelconque des revendications 1 à 13 et un second compartiment renferme une composition oxydante.

21. Nouveaux 4,5-diamino pyrazoles 3-substitués et leurs sels d'addition avec un acide, ayant pour formule : 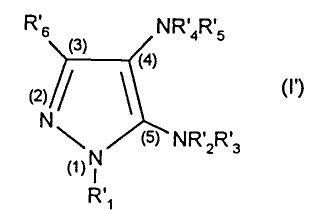 dans laquelle R'₁, R'₂, R'₃, R'₄, R'₅ et R'₆ ont les mêmes significations que celles indiquées précédemment pour les radicaux R₁, R₂, R₃, R₄, R₅ et R₆ dans la formule (I), avec les réserves suivantes :
(i) lorsque R'₁ représente un radical méthyle, et que R'₂, R'₄ et R'₅ représentent simultanément un atome d'hydrogène et que R'₃ représente un atome d'hydrogène ou un radical méthyle, alors R'₆ est différent d'un radical hydroxyméthyle, isobutyloxyméthyle, méthoxyéthyloxyméthyle, cyclohexyle, thiophène, pyridine, phényle ou phényle substitué par un radical méthyle ou par un radical trifluorométhyle ou par un atome de chlore ;
(ii) lorsque R'₁ représente un radical phényle non substitué, et que R'₂, R'₃, R'₄ et R'₅ représentent simultanément un atome d'hydrogène, alors R'₆ est différent d'un radical phényle non substitué ;
(iii) lorsque R'₁ représente un radical phényle non substitué, et que R'₆ représente un radical méthyle et que R'₂, R'₄ et R'₅ représentent simultanément un atome d'hydrogène, alors R'₃ est différent d'un atome d'hydrogène, d'un radical méthyle ou phényle non substitué ;
(iv) lorsque R'₁ représente un radical phényle non substitué, et que R'₆ représente un radical méthyle et que R'₄ et R'₅ représentent simultanément un atome d'hydrogène et que R'₂ représente un radical méthyle ou éthyle, alors R'₃ est différent d'un radical phényle non substitué ;
(v) lorsque R'₁ représente un radical phényle non substitué, et que R'₆ représente un radical méthyle et que R'₂, R'₃ et R'₅ représentent simultanément un atome d'hydrogène, alors R'₄ est différent d'un radical méthyle ;
(vi) lorsque R'₂, R'₃, R'₄ et R'₅ représentent simultanément un atome d'hydrogène, et que R'₆ représente un radical méthyle, alors R'₁ est différent d'un radical phényle substitué par un atome de chlore, par un radical trifluoroéthyle, nitro ou pyridyle ;
(vii) lorsque R'₁ représente un atome d'hydrogène, et que R'₆ représente un radical phényle ou un radical phényle substitué par un atome de chlore, par un radical méthyle ou méthoxy et que R'₂, R'₄ et R'₅ représentent simultanément un atome d'hydrogène, alors R'₃ est différent d'un atome d'hydrogène, d'un radical alkyle en C₁-C₄ ou phényle non substitué ;
(viii) lorsque R'₁, R'₂, R'₃, R'₄ et R'₅ représentent simultanément un atome d'hydrogène, alors R'₆ est différent d'un radical méthyle ;
(ix) lorsque R'₁ représente un radical β-hydroxyéthyle, et que R'₂, R'₃, R'₄ et R'₅ représentent simultanément un atome d'hydrogène, alors R'₆ est différent d'un radical méthyle ou phényle non substitué ;
(x) lorsque R'₄ représente un radical méthyle, et que R'₅ représente un radical phényle non substitué et que R'₂ et R'₃ représentent simultanément un atome d'hydrogène et que R'₁ représente un atome d'hydrogène ou un radical méthyle, alors R'₆ est différent d'un radical phényle non substitué ou d'un radical phényle substitué par un radical méthyle, éthyle, méthoxy ou par un atome de chlore ;
(xi) lorsque R'₁ représente un radical ter-butyle, et que R'₂, R'₃, R'₄ et R'₅ représentent simultanément un atome d'hydrogène, alors R'₆ est différent d'un radical méthyle ;
(xii) lorsque R'₁ représente un radical pyridyle, et que R'₂, R'₄ et R'₅ représentent simultanément un atome d'hydrogène et que R'₃ représente un atome d'hydrogène ou un radical méthyle, alors R'₆ est différent d'un radical méthyle ou phényle non substitué ;
(xiii) lorsque R'₁ représente un radical méthyle, éthyle ou 4-aminophényle, et que R'₂, R'₄ et R'₅ représentent simultanément un atome d'hydrogène et que R'₃ représente un atome d'hydrogène ou un radical phényle non substitué alors R'₆ est différent d'un radical méthyle ;
(xiv) lorsque R'₁ représente un radical isopropyle, et que R'₂, R'₄ et R'₅ représentent simultanément un atome d'hydrogène, alors au moins un des radicaux R'₃ et R'₆ est différent d'un radical méthyle ;
(xv) lorsque R'₁ représente un atome d'hydrogène ou un radical phényle non substitué, et que R'₂, R'₄ et R'₅ représentent simultanément un atome d'hydrogène et que R'₃ représente un radical benzyle ou un radical phényle substitué par un radical méthyle ou par un atome de chlore, alors R'₆ est différent d'un radical méthyle ou phényle non substitué.

22. Composés selon la revendication 21, caractérisés par le fait qu'ils sont choisis parmi :
- le 1-benzyl 4,5-diamino 3-méthyl pyrazole,
- le 4,5-diamino 1-(β-hydroxyéthyl) 3-(4'-méthoxyphényl) pyrazole,
- le 4,5-diamino 1-(β-hydroxyéthyl) 3-(4'-méthylphényl) pyrazole,
- le 4,5-diamino 1-(β-hydroxyéthyl) 3-(3'-méthylphényl) pyrazole,
- le 4,5-diamino 3-méthyl 1-isopropyl pyrazole,
- le 4,5-diamino 3-(4'-méthoxyphényl) 1-isopropyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 3-hydroxyméthyl 1-méthyl pyrazole,
- le 4,5-diamino1-éthyl 3-hydroxyméthyl pyrazole,
- le 4,5-diamino 3-hydroxyméthyl 1-isopropyl pyrazole,
- le 4,5-diamino 3-hydroxyméthyl 1-ter-butyl pyrazole,
- le 4,5-diamino 3-hydroxyméthyl 1-phényl pyrazole,
- le 4,5-diamino 3-hydroxyméthyl 1-(2'-méthoxyphényl) pyrazole,
- le 4,5-diamino 3-hydroxyméthyl 1-(3'-méthoxyphényl) pyrazole,
- le 4,5-diamino 3-hydroxyméthyl 1-(4'-méthoxyphényl) pyrazole,
- le 1-benzyl 4,5-diamino 3-hydroxyméthyl pyrazole,
- le 4,5-diamino 3-méthyl 1-(2'-méthoxyphényl) pyrazole,
- le 4,5-diamino 3-méthyl 1-(3'-méthoxyphényl) pyrazole,
- le 4,5-diamino 3-méthyl 1-(4'-méthoxyphényl) pyrazole,
- le 3-aminométhyl 4,5-diamino 1-méthyl pyrazole,
- le 3-aminométhyl 4,5-diamino 1-éthyl pyrazole,
- le 3-aminométhyl 4,5-diamino 1-isopropyl pyrazole,
- le 3-aminométhyl 4,5-diamino 1-ter-butyl pyrazole,
- le 4,5-diamino 3-diméthylaminométhyl 1-méthyl pyrazole,
- le 4,5-diamino 3-diméthylaminométhyl 1-éthyl pyrazole,
- le 4,5-diamino 3-diméthylaminométhyl 1-isopropyl pyrazole,
- le 4,5-diamino 3-diméthylaminométhyl 1-ter-butyl pyrazole,
- le 4,5-diamino 3-éthylaminométhyl 1-méthyl pyrazole,
- le 4,5-diamino 3-éthylaminométhyl 1-éthyl pyrazole,
- le 4,5-diamino 3-éthylaminométhyl 1-isopropyl pyrazole,
- le 4,5-diamino 3-éthylaminométhyl 1-ter-butyl pyrazole,
- le 4,5-diamino 3-méthylaminométhyl 1-méthyl pyrazole,
- le 4,5-diamino 3-méthylaminométhyl 1-isopropyl pyrazole,
- le 4,5-diamino 1-éthyl 3-méthylaminométhyl pyrazole,
- le 1-ter-butyl 4,5-diamino 3-méthylaminométhyl pyrazole,
- le 4,5-diamino 3-[(β-hydroxyéthyl)aminométhyl] 1-méthyl pyrazole,
- le 4,5-diamino 3-[(β-hydroxyéthyl)aminométhyl] 1-isopropyl pyrazole,
- le 4,5-diamino 1-éthyl 3-[(β-hydroxyéthyl)aminométhyl] pyrazole,
- le 1-ter-butyl 4,5-diamino 3-[(β-hydroxyéthyl)aminométhyl] pyrazole,
- le 4-amino 5-(β-hydroxyéthyl)amino 1,3-diméthyl pyrazole,
- le 4-amino 5-(β-hydroxyéthyl)amino 1-isopropyl 3-méthyl pyrazole,
- le 4-amino 5-(β-hydroxyéthyl)amino 1-éthyl 3-méthyl pyrazole,
- le 4-amino 5-(β-hydroxyéthyl)amino 1-ter-butyl 3-méthyl pyrazole,
- le 4-amino 5-(β-hydroxyéthyl)amino 1-phényl 3-méthyl pyrazole,
- le 4-amino 5-(β-hydroxyéthyl)amino 1-(2'-méthoxyphényl) 3-méthyl pyrazole,
- le 4-amino 5-(β-hydroxyéthyl)amino 1-(3'-méthoxyphényl) 3-méthyl pyrazole,
- le 4-amino 5-(β-hydroxyéthyl)amino 1-(4'-méthoxyphényl) 3-méthyl pyrazole,
- le 4-amino 5-(β-hydroxyéthyl)amino 1-benzyl 3-méthyl pyrazole,
- le 4-amino 1-éthyl 3-méthyl 5-méthylamino pyrazole,
- le 4-amino 1-ter-butyl 3-méthyl 5-méthylamino pyrazole,
- le 1-(4'-chlorobenzyl) 4,5-diamino 3-méthyl pyrazole,
- le 4,5-diamino 3-hydroxyméthyl 1-isopropyl pyrazole,
- le 4-amino 1-éthyl 3-méthyl 5-méthylamino pyrazole,
- le 4-amino 5-(2'-aminoéthyl)amino 1,3-diméthyl pyrazole,
et leurs sels d'addition avec un acide.

23. Composés selon la revendication 22, caractérisés par le fait qu'ils sont choisis parmi :
- le 1-benzyl 4,5-diamino 3-méthyl pyrazole,
- le 4,5-diamino 1-éthyl 3-méthyl pyrazole,
- le 4,5-diamino 1-éthyl 3-(4'-méthoxyphényl) pyrazole
- le 4,5-diamino 1-éthyl 3-hydroxyméthyl pyrazole,
- le 4,5-diamino 3-hydroxyméthyl 1-méthyl pyrazole,
- le 4,5-diamino 3-hydroxyméthyl 1-isopropyl pyrazole,
- le 4,5-diamino 3-méthyl 1-isopropyl pyrazole,
- le 4-amino 5-(2'-aminoéthyl)amino 1,3-diméthyl pyrazole,
et leurs sels d'addition avec un acide.

24. Procédé de préparation d'un composé de formule (I') selon l'une quelconque des revendications 21 à 23, dans lequel R'₆ représente un radical méthyle et R'₁ est différent d'un atome d'hydrogène, caractérisé par le fait qu'il consiste à faire réagir, dans une première étape, un 3-aminocrotononitrile avec une hydrazine monosubstituée, à une température supérieure à 90°C dans un solvant alcoolique puis, dans une deuxième étape, à nitroser le 5-aminopyrazole en position 4, par réaction avec un nitrite minéral ou organique pour donner un 5-amino 4-nitrosopyrazole, qui conduit, dans une troisième étape, par hydrogénation catalytique aux 4,5-diamino pyrazoles de formule (I') dans lesquels R'₆ représente un radical méthyle et R'₁ est différent d'un atome d'hydrogène.

25. Procédé de préparation d'un composé de formule (I') selon l'une quelconque des revendications 21 à 23, dans lequel R'₆ est différent d'un radical méthyle et R'₁ est différent d'un atome d'hydrogène, caractérisé par le fait qu'il consiste à faire réagir, dans une première étape, un β-cétoacétonitrile avec une hydrazine monosubstituée, à une température comprise entre 20 et 150°C, dans un solvant alcoolique, pour obtenir un 5-aminopyrazole qui est ensuite nitrosé en position 4, dans une deuxième étape, pour donner un 4-nitro 5-amino pyrazole qui est ensuite lui-même hydrogéné, dans une troisième étape, pour conduire aux 4,5-diamino pyrazoles de formule (I') dans lesquels R'₆ est différent d'un radical méthyle et R'₁ est différent d'un atome d'hydrogène.

26. Procédé de préparation d'un composé de formule (I') selon l'une quelconque des revendications 21 à 23, dans lequel R'₆ représente un radical à fort encombrement stérique, caractérisé par le fait qu'il consiste à faire réagir, dans une première étape, un β-cétoacétonitrile avec une hydrazine monosubstituée pour obtenir un 5-aminopyrazole qui est ensuite acétylé en position 5, dans une deuxième étape, pour conduire à un 5-acétylamino pyrazole qui est lui-même ensuite nitré en position 4 et désacétylé en position 5, dans une troisième étape, pour donner un 5-amino 4-nitro pyrazole qui est ensuite hydrogéné, dans une quatrième étape, pour conduire aux 4,5-diamino pyrazoles de formule (I') dans lesquels R'₆ représente un radical à fort encombrement stérique.

27. Procédé de préparation d'un composé de formule (I') selon l'une quelconque des revendications 21 à 23, dans lequel un des radicaux R'₂ ou R'₃ est différent d'un atome d'hydrogène, caractérisé par le fait qu'il consiste à faire réagir, dans une première étape, un β-cétoester avec une hydrazine, pour obtenir un 5-hydroxypyrazole en équilibre avec sa forme tautomère pyrazol-5-one, qui est ensuite nitré en position 4, dans une deuxième étape, puis chloré en position 5, dans une troisième étape, pour obtenir un 5-chloro 4-nitro pyrazole, qui conduit ensuite, dans une quatrième étape, en présence d'une amine primaire H₂N-R'₃ à un 5-amino 4-nitro pyrazole, puis dans une cinquième étape, par hydrogénation catalytique, aux 4,5-diamino pyrazoles de formule (I') dans lesquels un des radicaux R'₂ ou R'₃ est différent d'un atome d'hydrogène.

## Claims

1. Composition for the oxidation dyeing of keratin fibres, and in particular of human keratin fibres such as the hair, characterized in that it comprises, in a medium which is suitable for dyeing, at least one 3-substituted 4,5-diaminopyrazole of following formula (I) as oxidation base and/or at least one of the addition salts thereof with an acid: 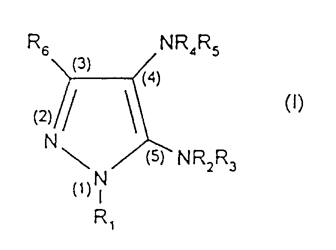 in which:
- R₁, R₂, R₃, R₄ and R₅ which may be identical or different, represent a hydrogen atom; a linear or branched C₁-C₆ alkyl radical; a C₂-C₄ hydroxyalkyl radical; a C₂-C₄ aminoalkyl radical; a phenyl radical; a phenyl radical substituted with a halogen atom or a C₁-C₄ alkyl, C₁-C₄ alkoxy, nitro, trifluoromethyl, amino or C₁-C₄ alkylamino radical; a benzyl radical; a benzyl radical substituted with a halogen atom or with a C₁-C₄ alkyl, C₁-C₄ alkoxy, methylenedioxy or amino radical; or a radical 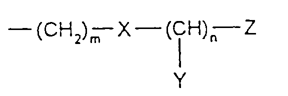 in which m and n are integers, which may be identical or different, between 1 and 3 inclusive, X represents an oxygen atom or the NH group, Y represents a hydrogen atom or a methyl radical and Z represents a methyl radical, a group OR or NRR' in which R and R', which may be identical or different, denote a hydrogen atom, a methyl radical or an ethyl radical,
it being understood that when R₂ represents a hydrogen atom, R₃ may then also represent an amino or C₁-C₄ alkylamino radical,
- R₆ represents a linear or branched C₁-C₆ alkyl radical; a C₁-C₄ hydroxyalkyl radical; a C₁-C₄ aminoalkyl radical; a (C₁-C₄)alkylamino(C₁-C₄)alkyl radical; a di(C₁-C₄)alkylamino(C₁-C₄)alkyl radical; a hydroxy(C₁-C₄)alkylamino(C₁-C₄)alkyl radical; a (C₁-C₄)alkoxymethyl radical; a phenyl radical; a phenyl radical substituted with a halogen atom or with a C₁-C₄ alkyl, C₁-C₄ alkoxy, nitro, trifluoromethyl, amino or C₁-C₄ alkylamino radical; a benzyl radical; a benzyl radical substituted with a halogen atom or with a C₁-C₄ alkyl, C₁-C₄ alkoxy, nitro, trifluoromethyl, amino or C₁-C₄ alkylamino radical; a heterocycle chosen from thiophene, furan and pyridine, or alternatively a radical -(CH₂)ₚ-O-(CH₂)_{q}-OR'', in which p and q are integers, which may be identical or different, between 1 and 3 inclusive and R'' represents a hydrogen atom or a methyl radical,
it being understood that, in the above formula (I):
- at least one of the radicals R₂, R₃, R₄ and R₅ represents a hydrogen atom,
- when R₂, or R₄, represents a substituted or unsubstituted phenyl radical or a benzyl radical or a radical 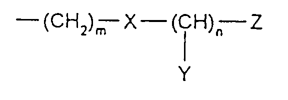 then R₃, or R₅, cannot represent any of these three radicals,
- when R₄ and R₅ simultaneously represent a hydrogen atom, then R₁ can form, with R₂ and R₃, a hexahydropyrimidine or tetrahydroimidazole heterocycle optionally substituted with a C₁-C₄ alkyl or 1,2,4-tetrazole radical,
- when R₂, R₃, R₄ and R₅ represent a hydrogen atom or a C₁-C₆ alkyl radical, then R₁ or R₆ may also represent a 2-, 3- or 4-pyridyl, 2- or 3-thienyl or 2- or 3-furyl heterocyclic residue optionally substituted with a methyl radical or alternatively a cyclohexyl radical.

2. Composition according to Claim 1, characterized in that the 3-substituted 4,5-diamino-pyrazoles of formula (I) are chosen from:
- 1-benzyl-4,5-diamino-3-methylpyrazole,
- 4,5-diamino-1-(β-hydroxyethyl)-3-(4'-methoxyphenyl)pyrazole,
- 4,5-diamino-1-(β-hydroxyethyl)-3-(4'-methylphenyl)pyrazole,
- 4,5-diamino-1-(β-hydroxyethyl)-3-(3'-methylphenyl)pyrazole,
- 4,5-diamino-3-methyl-1-isopropylpyrazole,
- 4,5-diamino-3-(4'-methoxyphenyl)-1-isopropyl-pyrazole,
- 4,5-diamino-1-ethyl-3-methylpyrazole,
- 4,5-diamino-1-ethyl-3-(4'-methoxyphenyl)pyrazole,
- 4,5-diamino-3-hydroxymethyl-1-methylpyrazole,
- 4,5-diamino-1-ethyl-3-hydroxymethylpyrazole,
- 4,5-diamino-3-hydroxymethyl-1-isopropylpyrazole,
- 4,5-diamino-3-hydroxymethyl-1-tert-butylpyrazole,
- 4,5-diamino-3-hydroxymethyl-1-phenylpyrazole,
- 4,5-diamino-3-hydroxymethyl-1-(2'-methoxyphenyl)pyrazole,
- 4,5-diamino-3-hydroxymethyl-1-(3'-methoxyphenyl)pyrazole,
- 4,5-diamino-3-hydroxymethyl-1-(4'-methoxyphenyl)pyrazole,
- 1-benzyl-4,5-diamino-3-hydroxymethylpyrazole,
- 4,5-diamino-3-methyl-1-(2'-methoxyphenyl)pyrazole,
- 4,5-diamino-3-methyl-1-(3'-methoxyphenyl)pyrazole,
- 4,5-diamino-3-methyl-1-(4'-methoxyphenyl)pyrazole,
- 3-aminomethyl-4,5-diamino-1-methylpyrazole,
- 3-aminomethyl-4,5-diamino-1-ethylpyrazole,
- 3-aminomethyl-4,5-diamino-1-isopropylpyrazole,
- 3-aminomethyl-4,5-diamino-1-tert-butylpyrazole,
- 4,5-diamino-3-dimethylaminomethyl-1-methylpyrazole,
- 4,5-diamino-3-dimethylaminomethyl-1-ethylpyrazole,
- 4,5-diamino-3-dimethylaminomethyl-1-isopropyl-pyrazole,
- 4,5-diamino-3-dimethylaminomethyl-1-tert-butylpyrazole,
- 4,5-diamino-3-ethylaminomethyl-1-methylpyrazole,
- 4,5-diamino-3-ethylaminomethyl-1-ethylpyrazole,
- 4,5-diamino-3-ethylaminomethyl-1-isopropylpyrazole,
- 4,5-diamino-3-ethylaminomethyl-1-tert-butylpyrazole,
- 4,5-diamino-3-methylaminomethyl-1-methylpyrazole,
- 4,5-diamino-3-methylaminomethyl-1-isopropylpyrazole,
- 4,5-diamino-1-ethyl-3-methylaminomethylpyrazole,
- 1-tert-butyl-4,5-diamino-3-methylaminomethyl-pyrazole,
- 4,5-diamino-3-[(β-hydroxyethyl)aminomethyl]-1-methylpyrazole,
- 4,5-diamino-3-[(β-hydroxyethyl)aminomethyl]-1-isopropylpyrazole,
- 4,5-diamino-1-ethyl-3-[(β-hydroxyethyl)aminomethyl]pyrazole,
- 1-tert-butyl-4,5-diamino-3-[(β-hydroxyethyl)aminomethyl]pyrazole,
- 4-amino-5-(β-hydroxyethyl)amino-1,3-dimethylpyrazole,
- 4-amino-5-(β-hydroxyethyl)amino-1-isopropyl-3-methylpyrazole,
- 4-amino-5-(β-hydroxyethyl)amino-1-ethyl-3-methylpyrazole,
- 4-amino-5-(β-hydroxyethyl)amino-1-tert-butyl-3-methylpyrazole,
- 4-amino-5-(β-hydroxyethyl)amino-1-phenyl-3-methylpyrazole,
- 4-amino-5-(β-hydroxyethyl)amino-1-(2-methoxyphenyl)-3-methylpyrazole,
- 4-amino-5-(β-hydroxyethyl)amino-1-(3-methoxyphenyl)-3-methylpyrazole,
- 4-amino-5-(β-hydroxyethyl)amino-1-(4-methoxyphenyl)-3-methylpyrazole,
- 4-amino-5-(β-hydroxyethyl)amino-1-benzyl-3-methylpyrazole,
- 4-amino-1-ethyl-3-methyl-5-methylaminopyrazole,
- 4-amino-1-tert-butyl-3-methyl-5-methylaminopyrazole,
- 4,5-diamino-1,3-dimethylpyrazole,
- 4,5-diamino-3-tert-butyl-1-methylpyrazole,
- 4,5-diamino-1-tert-butyl-3-methylpyrazole,
- 4,5-diamino-1-methyl-3-phenylpyrazole,
- 4,5-diamino-1-(β-hydroxyethyl)-3-methylpyrazole,
- 4,5-diamino-1-(β-hydroxyethyl)-3-phenylpyrazole,
- 4,5-diamino-1-methyl-3-(2'-chlorophenyl)pyrazole,
- 4,5-diamino-1-methyl-3-(4'-chlorophenyl)pyrazole,
- 4,5-diamino-1-methyl-3-(3'-trifluoromethylphenyl)pyrazole,
- 4,5-diamino-1,3-diphenylpyrazole,
- 4,5-diamino-3-methyl-1-phenylpyrazole,
- 4-amino-1,3-dimethyl-5-phenylaminopyrazole,
- 4-amino-1-ethyl-3-methyl-5-phenylaminopyrazole,
- 4-amino-1,3-dimethyl-5-methylaminopyrazole,
- 4-amino-3-methyl-1-isopropyl-5-methylaminopyrazole,
- 4-amino-3-isobutoxymethyl-1-methyl-5-methylaminopyrazole,
- 4-amino-3-methoxyethoxymethyl-1-methyl-5-methylaminopyrazole,
- 4-amino-3-hydroxymethyl-1-methyl-5-methylaminopyrazole,
- 4-amino-1,3-diphenyl-5-phenylaminopyrazole,
- 4-amino-3-methyl-5-methylamino-1-phenylpyrazole,
- 4-amino-1,3-dimethyl-5-hydrazinopyrazole,
- 5-amino-3-methyl-4-methylamino-1-phenylpyrazole,
- 5-amino-1-methyl-4-(N,N-methylphenyl)amino-3-(4'-chlorophenyl)pyrazole,
- 5-amino-3-ethyl-1-methyl-4-(N,N-methylphenyl)aminopyrazole,
- 5-amino-1-methyl-4-(N,N-methylphenyl)amino-3-phenylpyrazole,
- 5-amino-3-ethyl-4-(N,N-methylphenyl)aminopyrazole,
- 5-amino-4-(N,N-methylphenyl)amino-3-phenylpyrazole,
- 5-amino-4-(N,N-methylphenyl)amino-3-(4'-methylphenyl)pyrazole,
- 5-amino-3-(4'-chlorophenyl)-4-(N,N-methylphenyl)aminopyrazole,
- 5-amino-3-(4'-methoxyphenyl)-4-(N,N-methylphenyl)aminopyrazole,
- 4-amino-5-methylamino-3-phenylpyrazole,
- 4-amino-5-ethylamino-3-phenylpyrazole,
- 4-amino-5-ethylamino-3-(4'-methylphenyl)pyrazole,
- 4-amino-3-phenyl-5-propylaminopyrazole,
- 4-amino-5-butylamino-3-phenylpyrazole,
- 4-amino-3-phenyl-5-phenylaminopyrazole,
- 4-amino-5-benzylamino-3-phenylpyrazole,
- 4-amino-5-(4'-chlorophenyl)amino-3-phenylpyrazole,
- 4-amino-3-(4'-chlorophenyl)-5-phenylaminopyrazole,
- 4-amino-3-(4'-methoxyphenyl)-5-phenylaminopyrazole,
- 1-(4'-chlorobenzyl)-4,5-diamino-3-methylpyrazole,
- 4,5-diamino-3-hydroxymethyl-1-isopropylpyrazole,
- 4-amino-1-ethyl-3-methyl-5-methylaminopyrazole,
- 4-amino-5-(2'-aminoethyl)amino-1,3-dimethylpyrazole,
and the addition salts thereof with an acid.

3. Composition according to Claim 2, characterized in that the 3-substituted 4,5-diamino- pyrazoles of formula (I) are chosen from:
- 4,5-diamino-1,3-dimethylpyrazole,
- 4,5-diamino-3-methyl-1-phenylpyrazole,
- 4,5-diamino-1-methyl-3-phenylpyrazole,
- 4-amino-1,3-dimethyl-5-hydrazinopyrazole,
- 1-benzyl-4,5-diamino-3-methylpyrazole,
- 4,5-diamino-3-tert-butyl-1-methylpyrazole,
- 4,5-diamino-1-tert-butyl-3-methylpyrazole,
- 4,5-diamino-1-(β-hydroxyethyl)-3-methylpyrazole,
- 4,5-diamino-1-ethyl-3-methylpyrazole,
- 4,5-diamino-1-ethyl-3-(4'-methoxyphenyl)pyrazole,
- 4,5-diamino-1-ethyl-3-hydroxymethylpyrazole,
- 4,5-diamino-3-hydroxymethyl-1-methylpyrazole,
- 4,5-diamino-3-hydroxymethyl-1-isopropylpyrazole,
- 4,5-diamino-3-methyl-1-isopropylpyrazole,
- 4-amino-5-(2'-aminoethyl)amino-1,3-dimethylpyrazole,
and the addition salts thereof with an acid.

4. Composition according to any one of the preceding claims, characterized in that the 3-substituted 4,5-diaminopyrazole(s) of formula (I) represent from 0.0005 to 12 % by weight relative to the total weight of the dye composition.

5. Composition according to Claim 4, characterized in that the 3-substituted 4,5-diaminopyrazole(s) of formula (I) represent from 0.005 to 6 % by weight relative to the total weight of the dye composition.

6. Composition according to any one of the preceding claims, characterized in that the medium which is suitable for dyeing (or the support) consists of water or of a mixture of water and at least one organic solvent chosen from C₁-C₄ lower alkanols, glycerol, glycols and glycol ethers, aromatic alcohols, similar products and mixtures thereof.

7. Composition according to any one of the preceding claims, characterized in that it has a pH of between 3 and 12.

8. Composition according to any one of the preceding claims, characterized in that it contains at least one additional oxidation base chosen from para-phenylenediamines, bis(phenyl)alkylenediamines, para-aminophenols, ortho-aminophenols and heterocyclic bases other than the 3-substituted 4,5-diaminopyrazoles of formula (I).

9. Composition according to Claim 8, characterized in that the additional oxidation base or bases represent from 0.0005 to 12 % by weight relative to the total weight of the dye composition.

10. Composition according to any one of the preceding claims, characterized in that it contains at least one coupler and/or at least one direct dye.

11. Composition according to Claim 10, characterized in that the coupler or couplers are chosen from meta-phenylenediamines, meta-aminophenols, meta-diphenols and heterocyclic couplers, and the addition salts thereof with an acid.

12. Composition according to Claim 10 or 11, characterized in that the coupler or couplers represent from 0.0001 to 10 % by weight relative to the total weight of the dye composition.

13. Composition according to any one of the preceding claims, characterized in that the addition salts with an acid are chosen from the hydrochlorides, hydrobromides, sulphates, tartrates, lactates and acetates.

14. Process for dyeing keratin fibres, and in particular human keratin fibres such as the hair, characterized in that at least one dye composition as defined in any one of Claims 1 to 13 is applied to these fibres, for a period which is sufficient to develop the desired coloration, either in air or with the aid of an oxidizing agent.

15. Process according to Claim 14, characterized in that the coloration is developed merely by contact with atmospheric oxygen.

16. Process according to Claim 15, characterized in that the coloration is developed merely by contact with atmospheric oxygen, in the presence of oxidation catalysts.

17. Process according to Claim 16, characterized in that the oxidation catalysts are metal salts.

18. Process according to Claim 14, characterized in that the colour is developed at acidic, neutral or alkaline pH using an oxidizing agent which is added, only at the time of use, to the dye composition or which is present in an oxidizing composition that is applied simultaneously or sequentially in a separate manner.

19. Process according to Claim 18, characterized in that the oxidizing agent is chosen from hydrogen peroxide, urea peroxide, alkali metal bromates and persalts such as perborates and persulphates.

20. Multicompartment device or multicompartment dyeing "kit", a first compartment of which contains a dye composition as defined in any one of Claims 1 to 13, and a second compartment of which contains an oxidizing composition.

21. Novel 3-substituted 4,5-diaminopyrazoles and the addition salts thereof with an acid, having the formula: 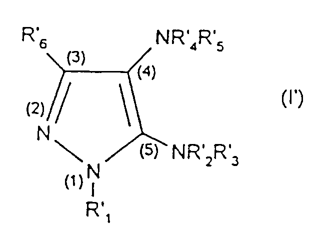 in which R'₁, R'₂, R'₃, R'₄, R'₅ and R'₆ have the same meanings as those indicated above for the radicals R₁, R₂, R₃, R₄, R₅ and R₆ in the formula (I), with the following provisos:
(i) when R'₁ represents a methyl radical, and when R'₂, R'₄ and R'₅ simultaneously represent a hydrogen atom, and when R'₃ represents a hydrogen atom or a methyl radical, then R'₆ is other than a hydroxymethyl, isobutyloxymethyl, methoxyethyloxymethyl, cyclohexyl, thiophene, pyridine or phenyl radical or phenyl radical substituted with a methyl radical or with a trifluoromethyl radical or with a chlorine atom;
(ii) when R'₁ represents an unsubstituted phenyl radical and when R'₂, R'₃, R'₄ and R'₅ simultaneously represent a hydrogen atom, then R'₆ is other than an unsubstituted phenyl radical;
(iii) when R'₁ represents an unsubstituted phenyl radical, and when R'₆ represents a methyl radical, and when R'₂, R'₄ and R'₅ simultaneously represent a hydrogen atom, then R'₃ is other than a hydrogen atom, a methyl radical or an unsubstituted phenyl radical;
(iv) when R'₁ represents an unsubstituted phenyl radical, and when R'₆ represents a methyl radical, and when R'₄ and R'₅ simultaneously represent a hydrogen atom, and when R'₂ represents a methyl or ethyl radical, then R'₃ is other than an unsubstituted phenyl radical;
(v) when R'₁ represents an unsubstituted phenyl radical, and when R'₆ represents a methyl radical, and R'₂, R'₃ and R'₅ simultaneously represent a hydrogen atom, then R'₄ is other than a methyl radical;
(vi) when R'₂, R'₃, R'₄ and R'₅ simultaneously represent a hydrogen atom, and when R'₆ represents a methyl radical, then R'₁ is other than a phenyl radical substituted with a chlorine atom or with a trifluoroethyl, nitro or pyridyl radical;
(vii) when R'₁ represents a hydrogen atom, and when R'₆ represents a phenyl radical or a phenyl radical substituted with a chlorine atom or with a methyl or methoxy radical, and when R'₂, R'₄ and R'₅ simultaneously represent a hydrogen atom, then R'₃ is other than a hydrogen atom or a C₁-C₄ alkyl or unsubstituted phenyl radical;
(viii) when R'₁, R'₂, R'₃, R'₄ and R'₅ simultaneously represent a hydrogen atom, then R'₆ is other than a methyl radical;
(ix) when R'₁ represents a β-hydroxyethyl radical, and when R'₂, R'₃, R'₄ and R'₅ simultaneously represent a hydrogen atom, then R'₆ is other than a methyl or unsubstituted phenyl radical;
(x) when R'₄ represents a methyl radical, and when R'₅ represents an unsubstituted phenyl radical, and when R'₂ and R'₃ simultaneously represent a hydrogen atom, and when R'₁ represents a hydrogen atom or a methyl radical, then R'₆ is other than an unsubstituted phenyl radical or a phenyl radical substituted with a methyl, ethyl or methoxy radical or with a chlorine atom;
(xi) when R'₁ represents a tert-butyl radical, and when R'₂, R'₃, R'₄ and R'₅ simultaneously represent a hydrogen atom, then R'₆ is other than a methyl radical;
(xii) when R'₁ represents a pyridyl radical, and when R'₂, R'₄ and R'₅ simultaneously represent a hydrogen atom, and when R'₃ represents a hydrogen atom or a methyl radical, then R'₆ is other than a methyl or unsubstituted phenyl radical;
(xiii) when R'₁ represents a methyl, ethyl or 4-aminophenyl radical, and when R'₂, R'₄ and R'₅ simultaneously represent a hydrogen atom, and when R'₃ represents a hydrogen atom or an unsubstituted phenyl radical, then R'₆ is other than a methyl radical;
(xiv) when R'₁ represents an isopropyl radical, and when R'₂, R'₄ and R'₅ simultaneously represent a hydrogen atom, then at least one of the radicals R'₃ and R'₆ is other than a methyl radical;
(xv) when R'₁ represents a hydrogen atom or an unsubstituted phenyl radical, and when R'₂, R'₄ and R'₅ simultaneously represent a hydrogen atom, and when R'₃ represents a benzyl radical or a phenyl radical substituted with a methyl radical or with a chlorine atom, then R'₆ is other than a methyl or unsubstituted phenyl radical.

22. Compounds according to Claim 21, characterized in that they are chosen from:
- 1-benzyl-4,5-diamino-3-methylpyrazole,
- 4,5-diamino-1-(β-hydroxyethyl)-3-(4'-methoxyphenyl)pyrazole,
- 4,5-diamino-1-(β-hydroxyethyl)-3-(4'-methylphenyl)pyrazole,
- 4,5-diamino-1-(β-hydroxyethyl)-3-(3'-methylphenyl)pyrazole,
- 4,5-diamino-3-methyl-1-isopropylpyrazole,
- 4,5-diamino-3-(4'-methoxyphenyl)-1-isopropyl-pyrazole,
- 4,5-diamino-1-ethyl-3-methylpyrazole,
- 4,5-diamino-1-ethyl-3-(4'-methoxyphenyl)pyrazole,
- 4,5-diamino-3-hydroxymethyl-1-methylpyrazole,
- 4,5-diamino-1-ethyl-3-hydroxymethylpyrazole,
- 4,5-diamino-3-hydroxymethyl-1-isopropylpyrazole,
- 4,5-diamino-3-hydroxymethyl-1-tert-butylpyrazole,
- 4,5-diamino-3-hydroxymethyl-1-phenylpyrazole,
- 4,5-diamino-3-hydroxymethyl-1-(2'-methoxyphenyl)pyrazole,
- 4,5-diamino-3-hydroxymethyl-1-(3'-methoxyphenyl)pyrazole,
- 4,5-diamino-3-hydroxymethyl-1-(4'-methoxyphenyl)pyrazole,
- 1-benzyl-4,5-diamino-3-hydroxymethylpyrazole,
- 4,5-diamino-3-methyl-1-(2'-methoxyphenyl)pyrazole,
- 4,5-diamino-3-methyl-1-(3'-methoxyphenyl)pyrazole,
- 4,5-diamino-3-methyl-1-(4'-methoxyphenyl)pyrazole,
- 3-aminomethyl-4,5-diamino-1-methylpyrazole,
- 3-aminomethyl-4,5-diamino-1-ethylpyrazole,
- 3-aminomethyl-4,5-diamino-1-isopropylpyrazole,
- 3-aminomethyl-4,5-diamino-1-tert-butylpyrazole,
- 4,5-diamino-3-dimethylaminomethyl-1-methylpyrazole,
- 4,5-diamino-3-dimethylaminomethyl-1-ethylpyrazole,
- 4,5-diamino-3-dimethylaminomethyl-1-isopropyl-pyrazole,
- 4,5-diamino-3-dimethylaminomethyl-1-tert-butylpyrazole,
- 4,5-diamino-3-ethylaminomethyl-1-methylpyrazole,
- 4,5-diamino-3-ethylaminomethyl-1-ethylpyrazole,
- 4,5-diamino-3-ethylaminomethyl-1-isopropylpyrazole,
- 4,5-diamino-3-ethylaminomethyl-1-tert-butylpyrazole,
- 4,5-diamino-3-methylaminomethyl-1-methylpyrazole,
- 4,5-diamino-3-methylaminomethyl-1-isopropylpyrazole,
- 4,5-diamino-1-ethyl-3-methylaminomethylpyrazole,
- 1-tert-butyl-4,5-diamino-3-methylaminomethyl-pyrazole,
- 4,5-diamino-3-[(β-hydroxyethyl)aminomethyl]-1-methylpyrazole,
- 4,5-diamino-3-[(β-hydroxyethyl)aminomethyl]-1-isopropylpyrazole,
- 4,5-diamino-1-ethyl-3-[(β-hydroxyethyl)aminomethyl]pyrazole,
- 1-tert-butyl-4,5-diamino-3-[(β-hydroxyethyl)aminomethyl]pyrazole,
- 4-amino-5-(β-hydroxyethyl)amino-1,3-dimethylpyrazole,
- 4-amino-5-(β-hydroxyethyl)amino-1-isopropyl-3-methylpyrazole,
- 4-amino-5-(β-hydroxyethyl)amino-1-ethyl-3-methylpyrazole,
- 4-amino-5-(β-hydroxyethyl)amino-1-tert-butyl-3-methylpyrazole,
- 4-amino-5-(β-hydroxyethyl)amino-1-phenyl-3-methylpyrazole,
- 4-amino-5-(β-hydroxyethyl)amino-1-(2'-methoxyphenyl)-3-methylpyrazole,
- 4-amino-5-(β-hydroxyethyl)amino-1-(3'-methoxyphenyl)-3-methylpyrazole,
- 4-amino-5-(β-hydroxyethyl)amino-1-(4'-methoxyphenyl)-3-methylpyrazole,
- 4-amino-5-(β-hydroxyethyl)amino-1-benzyl-3-methylpyrazole,
- 4-amino-1-ethyl-3-methyl-5-methylaminopyrazole,
- 4-amino-1-tert-butyl-3-methyl-5-methylaminopyrazole,
- 1-(4'-chlorobenzyl)-4,5-diamino-3-methylpyrazole,
- 4,5-diamino-3-hydroxymethyl-1-isopropylpyrazole,
- 4-amino-1-ethyl-3-methyl-5-methylaminopyrazole,
- 4-amino-5-(2'-aminoethyl)amino-1,3-dimethylpyrazole,
and the addition salts thereof with an acid.

23. Compounds according to Claim 22, characterized in that they are chosen from:
- 1-benzyl-4,5-diamino-3-methylpyrazole,
- 4,5-diamino-1-ethyl-3-methylpyrazole,
- 4,5-diamino-1-ethyl-3-(4'-methoxyphenyl)pyrazole,
- 4,5-diamino-1-ethyl-3-hydroxymethylpyrazole,
- 4,5-diamino-3-hydroxymethyl-1-methylpyrazole,
- 4,5-diamino-3-hydroxymethyl-1-isopropylpyrazole,
- 4,5-diamino-3-methyl-1-isopropylpyrazole,
- 4-amino-5-(2'-aminoethyl)amino-1,3-dimethyl-pyrazole,
and the addition salts thereof with an acid.

24. Process for the preparation of a compound of formula (I') according to any one of Claims 21 to 23, in which R'₆ represents a methyl radical and R'₁ is other than a hydrogen atom, characterized in that it consists in reacting, in a first step, a 3-aminocrotononitrile with a monosubstituted hydrazine, at a temperature of above 90°C in an alcoholic solvent, followed, in a second step, in nitrosating the 5-aminopyrazole in the 4-position, by reaction with an inorganic or organic nitrite to give a 5-amino-4-nitrosopyrazole, which leads, in a third step, by catalytic hydrogenation, to the 4,5-diaminopyrazoles of formula (I') in which R'₆ represents a methyl radical and R'₁ is other than a hydrogen atom.

25. Process for the preparation of a compound of formula (I') according to any one of Claims 21 to 23, in which R'₆ is other than a methyl radical and R'₁ is other than a hydrogen atom, characterized in that it consists in reacting, in a first step, a β-keto acetonitrile with a monosubstituted hydrazine, at a temperature of between 20 and 150°C, in an alcoholic solvent, in order to obtain a 5-aminopyrazole, which is then nitrosated in the 4-position, in a second step, to give a 4-nitro-5-aminopyrazole which is itself then hydrogenated, in a third step, to lead to the 4,5-diaminopyrazoles of formula (I') in which R'₆ is other than a methyl radical and R'₁ is other than a hydrogen atom.

26. Process for the preparation of a compound of formula (I') according to any one of Claims 21 to 23, in which R'₆ represents a radical of high steric bulk, characterized in that it consists in reacting, in a first step, a β-keto acetonitrile with a monosubstituted hydrazine in order to obtain a 5-aminopyrazole, which is then acetylated in the 5-position, in a second step, to lead to a 5-acetylaminopyrazole, which is itself then nitrated in the 4-position and deacetylated in the 5-position, in a third step, to give a 5-amino-4-nitropyrazole, which is subsequently hydrogenated, in a fourth step, to lead to the 4,5-diaminopyrazoles of formula (I') in which R'₆ represents a radical of high steric bulk.

27. Process for the preparation of a compound of formula (I') according to any one of Claims 21 to 23, in which one of the radicals R'₂ and R'₃ is other than a hydrogen atom, characterized in that it consists in reacting, in a first step, a β-keto ester with a hydrazine, in order to obtain a 5-hydroxypyrazole in equilibrium with its 5-pyrazolone tautomeric form, which is then nitrated in the 4-position, in a second step, and then chlorinated in the 5-position, in a third step, in order to obtain a 5-chloro-4-nitropyrazole, which then leads, in a fourth step, in the presence of a primary amine H₂N-R'₃, to a 5-amino-4-nitropyrazole, followed, in a fifth step, by catalytic hydrogenation, to the 4,5-diaminopyrazoles of formula (I') in which one of the radicals R'₂ or R'₃ is other than a hydrogen atom.

## Patentansprüche

1. Zusammensetzung zum oxidativen Färben von Keratinfasern und insbesondere von menschlichen Keratinfasern, wie dem Haar,
**dadurch gekennzeichnet, daß**
sie in einem zum Färben geeigneten Medium mindestens ein in 3-Stellung substituiertes 4,5-Diaminopyrazol der folgenden Formel (I) als Oxidationsbase und/oder mindestens eines der Additionssalze dieser Verbindungen mit einer Säure enthält: 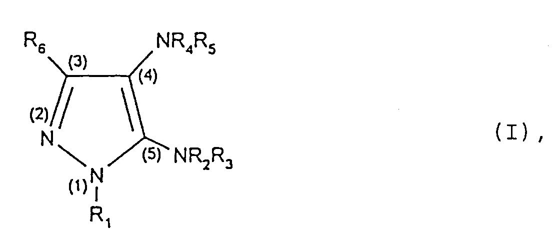 worin bedeuten:
- R₁, R₂, R₃, R₄ und R₅, die identisch oder voneinander verschieden sind, Wasserstoff, eine geradkettige oder verzweigte C₁₋₆-Alkylgruppe, C₂₋₄-Hydroxyalkyl, C₂₋₄-Aminoalkyl, Phenyl, Phenyl, das mit Halogen, C₁₋₄-Alkyl, C₁₋₄-Alkoxy, Nitro, Trifluormethyl, Amino oder C₁₋₄-Alkylamino substituiert ist, Benzyl, Benzyl, das mit Halogen, C₁₋₄-Alkyl, C₁₋₄-Alkoxy, Methylendioxy oder Amino substituiert ist, oder eine Gruppe 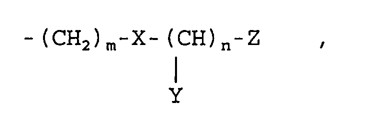 worin m und n ganze Zahlen bedeuten, die identisch oder voneinander verschieden sind und im Bereich von 1 bis einschließlich 3 liegen, X Sauerstoff oder eine Gruppe NH, Y Wasserstoff oder Methyl und Z Methyl, eine Gruppe OR oder NRR' bedeutet, worin R und R', die identisch oder voneinander verschieden sein können, Wasserstoff, Methyl oder Ethyl bedeuten,
mit der Maßgabe, daß R₃ auch eine Gruppe Amino oder C₁₋₄-Alkylamino bedeuten kann, wenn R₂ Wasserstoff bedeutet,
- R₆ eine geradkettige oder verzweigte C₁₋₆-Alkylgruppe, C₁₋₄-Hydroxyalkyl, C₁₋₄-Aminoalkyl, C₁₋₄-Alkyl-C₁₋₄-Aminoalkyl, C₁₋₄-Dialkyl-C₁₋₄-Aminoalkyl, C₁₋₄-Hydroxyalkyl-C₁₋₄-Aminoalkyl, C₁₋₄-Alkoxymethyl, Phenyl, Phenyl, das mit Halogen, C₁₋₄-Alkyl, C₁₋₄-Alkoxy, Nitro, Trifluormethyl, Amino oder C₁₋₄-Alkylamino substituiert ist, Benzyl, Benzyl, das mit Halogen, C₁₋₄-Alkyl, C₁₋₄-Alkoxy, Nitro, Trifluormethyl, Amino oder C₁₋₄-Alkylamino substituiert ist, einen Heterocyclus, der unter Thiophen, Furan und Pyridin ausgewählt ist, oder auch eine Gruppe -(CH₂)ₚ-O-(CH₂)_{q}-OR'', worin p und q ganze Zahlen bedeuten, die identisch oder voneinander verschieden sind und im Bereich von 1 bis einschließlich 3 liegen, und R'' Wasserstoff oder Methyl bedeutet, mit der Maßgabe, daß in der oben genannten Formel (I):
- mindestens eine der Gruppen R₂, R₃, R₄ und R₅ Wasserstoff bedeutet,
- wenn R₂ bzw. R₄ eine substituierte oder unsubstituierte Phenylgruppe oder eine Benzylgruppe oder eine Gruppe 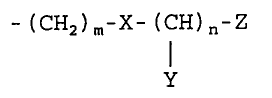 bedeuten, R₃ bzw. R₅ keine dieser drei Gruppen bedeuten kann,
- wenn R₄ und R₅ gleichzeitig Wasserstoff bedeuten, R₁ mit R₂ und R₃ einen Hexahydropyrimidin- oder Tetrahydroimidazol-Heterocyclus bilden kann, der gegebenenfalls mit C₁₋₄-Alkyl oder 1,2,4-Tetrazol substituiert sein kann,
- wenn R₂, R₃, R₄ und R₅ Wasserstoff oder C₁₋₆-Alkyl bedeuten, R₁ oder R₆ auch eine heterocyclische Gruppe 2-, 3- oder 4-Pyridyl, 2- oder 3-Thienyl oder 2- oder 3-Furyl bedeuten kann, die gegebenenfalls mit Methyl oder auch Cyclohexyl substituiert sein können.

2. Zusammensetzung nach Anspruch 1, dadurch gekennzeichnet, daß die in 3-Stellung substituierten 4,5-Diaminopyrazole der Formel (I) ausgewählt sind unter:
- 1-Benzyl-4,5-diamino-3-methyl-pyrazol,
- 4,5-Diamino-1-(β-hydroxyethyl)-3-(4'-methoxyphenyl)-pyrazol,
- 4,5-Diamino-1-(β-hydroxyethyl)-3-(4'-methylphenyl)-pyrazol,
- 4,5-Diamino-1-(β-hydroxyethyl)-3-(3'-methylphenyl)-pyrazol,
- 4,5-Diamino-3-methyl-1-isopropyl-pyrazol,
- 4,5-Diamino-3-(4'-methoxyphenyl)-1-isopropyl-pyrazol,
- 4,5-Diamino-1-ethyl-3-methyl-pyrazol,
- 4,5-Diamino-1-ethyl-3-(4'-methoxyphenyl)-pyrazol,
- 4,5-Diamino-3-hydroxymethyl-1-methyl-pyrazol,
- 4,5-Diamino-1-ethyl-3-hydroxymethyl-pyrazol,
- 4,5-Diamino-3-hydroxymethyl-1-isopropyl-pyrazol,
- 4,5-Diamino-3-hydroxymethyl-1-*tert*.-butyl-pyrazol,
- 4,5-Diamino-3-hydroxymethy1-1-phenyl-pyrazol,
- 4,5-Diamino-3-hydroxymethyl-1-(2'-methoxyphenyl)-pyrazol,
- 4,5-Diamino-3-hydroxymethyl-1-(3'-methoxyphenyl)-pyrazol,
- 4,5-Diamino-3-hydroxymethyl-1-(4'-methoxyphenyl)-pyrazol,
- 1-Benzyl-4,5-diamino-3-hydroxmethyl-pyrazol,
- 4,5-Diamino-3-methyl-1-(2'-methoxyphenyl)-pyrazol,
- 4,5-Diamino-3-methyl-1-(3'-methoxyphenyl)-pyrazol,
- 4,5-Diamino-3-methyl-1-(4'-methoxyphenyl)-pyrazol,
- 3-Aminomethyl-4,5-diamino-1-methyl-pyrazol,
- 3-Aminomethyl-4,5-diamino-1-ethyl-pyrazol,
- 3-Aminomethyl-4,5-diamino-1-isopropyl-pyrazol,
- 3-Aminomethyl-4,5-diamino-1-*tert*.-butyl-pyrazol,
- 4,5-Diamino-3-dimethylaminomethyl-1-methyl-pyrazol,
- 4,5-Diamino-3-dimethylaminomethyl-1-ethyl-pyrazol,
- 4,5-Diamino-3-dimethylaminomethyl-1-isopropyl-pyrazol,
- 4,5-Diamino-3-dimethylaminomethyl-1-*tert*.-butyl-pyrazol,
- 4,5-Diamino-3-ethylaminomethyl-1-methyl-pyrazol,
- 4,5-Diamino-3-ethylaminomethyl-1-ethyl-pyrazol,
- 4,5-Diamino-3-ethylaminomethyl-1-isopropyl-pyrazol,
- 4,5-Diamino-3-ethylaminomethyl-1-*tert*.-butyl-pyrazol,
- 4,5-Diamino-3-methylaminomethyl-1-methyl-pyrazol,
- 4,5-Diamino-3-methylaminomethyl-1-methyl-pyrazol,
- 4,5-Diamino-1-ethyl-3-methylaminomethyl-pyrazol,
- 1-*tert*.-butyl-4,5-diamino-3-methylaminomethyl-pyrazol,
- 4,5-Diamino-3-[(β-hydroxyethyl)aminomethyl]-1-methyl-pyrazol,
- 4,5-Diamino-3-[(β-hydroxyethyl)aminomethyl]-1-isopropylpyrazol,
- 4,5-Diamino-1-ethyl-3-[(β-hydroxyethyl)aminomethyl]-pyrazol,
- 1-*tert*.-Butyl-4,5-Diamino-3-[(β-hydroxyethyl)aminomethyl]pyrazol,
- 4-Amino-5-(β-hydroxyethyl)amino-1,3-dimethyl-pyrazol,
- 4-Amino-5-(β-hydroxyethyl)amino-1-isopropyl-3-methyl-pyrazol,
- 4-Amino-5-(β-hydroxyethyl)amino-1-ethyl-3-methyl-pyrazol,
- 4-Amino-5-(β-hydroxyethyl)amino-1-*tert*.-butyl-3-methyl-pyrazol,
- 4-Amino-5-(β-hydroxyethyl)amino-1-phenyl-3-methyl-pyrazol,
- 4-Amino-5-(β-hydroxyethyl)amino-1-(2-methoxyphenyl)-3-methyl-pyrazol,
- 4-Amino-5-(β-hydroxyethyl)amino-1-(3-methoxyphenyl)-3-methyl-pyrazol,
- 4-Amino-5-(β-hydroxyethyl)amino-1-(4-methoxyphenyl)-3-methyl-pyrazol,
- 4-Amino-5-(β-hydroxyethyl)amino-1-benzyl-3-methyl-pyrazol,
- 4-Amino-1-ethyl-3-methyl-5-methylamino-pyrazol,
- 4-Amino-1-*tert*.-butyl-3-methyl-5-methylamino-pyrazol,
- 4,5-Diamino-1,3-dimethyl-pyrazol,
- 4,5-Diamino-3-*tert*.-butyl-1-methyl-pyrazol,
- 4,5-Diamino-1-*tert*.-butyl-3-methyl-pyrazol,
- 4,5-Diamino-1-methyl-3-phenyl-pyrazol,
- 4,5-Diamino-1-(β-hydroxyethyl)-3-methyl-pyrazol,
- 4,5-Diamino-1-(β-hydroxyethyl)-3-phenyl-pyrazol,
- 4,5-Diamino-1-methyl-3-(2'-chlorphenyl)-pyrazol,
- 4,5-Diamino-1-methyl-3-(4'-chlorphenyl)-pyrazol,
- 4,5-Diamlno-1-methyl-3-(3'-trifluormethylphenyl)-pyrazol,
- 4,5-Diamino-1,3-diphenyl-pyrazol,
- 4,5-Diamino-3-methyl-1-phenyl-pyrazol,
- 4-Amino-1,3-dimethyl-5-phenylamino-pyrazol,
- 4-Amino-1-ethyl-3-methyl-5-phenylamino-pyrazol,
- 4-Amino-1,3-dimethyl-5-methylamino-pyrazol,
- 4-Amino-3-methyl-1-isopropyl-5-methylamino-pyrazol,
- 4-Amino-3-isobutoxymethyl-1-methyl-5-methylamino-pyrazol,
- 4-Amino-3-methoxyethoxymethyl-1-methyl-5-methylamino-pyrazol,
- 4-Amino-3-hydroxymethyl-1-methyl-5-methylamino-pyrazol,
- 4-Amino-1,3-diphenyl-5-phenylamino-pyrazol,
- 4-Amino-3-methyl-5-methylamino-1-phenyl-pyrazol,
- 4-Amino-1,3-dimethyl-5-hydrazino-pyrazol,
- 5-Amino-3-methyl-4-methylamino-1-phenyl-pyrazol,
- 5-Amino-1-methyl-4-(N,N-methylphenyl)amino-3-(4'-chlorphenyl)-pyrazol,
- 5-Amino-3-ethyl-1-methyl-4-(N,N-methylphenyl)amino-pyrazol,
- 5-Amino-1-methyl-4-(N,N-methylphenyl)amino-3-phenyl-pyrazol,
- 5-Amino-3-ethyl-4-(N,N-methylphenyl)amino-pyrazol,
- 5-Amino-4-(N,N-methylphenyl)amino-3-phenyl-pyrazol,
- 5-Amino-4-(N,N-methylphenyl)amino-3-(4'-methylphenyl)pyrazol,
- 5-Amino-3-(4'-chlorphenyl)-4-(N,N-methylphenyl)amino-pyrazol,
- 5-Amino-3-(4'-methoxyphenyl)-4-(N,N-methylphenyl)amino-pyrazol,
- 4-Amino-5-methylamino-3-phenyl-pyrazol,
- 4-Amino-5-ethylamino-3-phenyl-pyrazol,
- 4-Amino-5-ethylamino-3-(4'-methylphenyl)-pyrazol,
- 4-Amino-3-phenyl-5-propylamino-pyrazol,
- 4-Amino-5-butylamino-3-phenyl-pyrazol,
- 4-Amino-3-phenyl-5-phenylamino-pyrazol,
- 4-Amino-5-benzylamino-3-phenyl-pyrazol,
- 4-Amino-5-(4'-chlorphenyl)amino-3-phenyl-pyrazol,
- 4-Amino-3-(4'-chlorphenyl)-5-phenylamino-pyrazol,
- 4-Amino-3-(4'-methoxphenyl)-5-phenylamino-pyrazol,
- 1-(4'-Chlorbenzyl)-4,5-diamino-3-methyl-pyrazol,
- 4,5-Diamino-3-hydroxymethyl-1-isopropyl-pyrazol,
- 4-Amino-1-ethyl-3-methyl-5-methylamino-pyrazol,
- 4-Amino-5-(2'-aminoethyl)amino-1,3-dimethyl-pyrazol,
und den Additionssalzen dieser Verbindungen mit einer Säure.

3. Zusammensetzung nach Anspruch 2, dadurch gekennzeichnet, daß die in 3-Stellung substituierten 4,5-Diaminopyrazole der Formel (I) ausgewählt sind unter:
- 4,5-Diamino-1,3-dimethyl-pyrazol,
- 4,5-Diamino-3-methyl-1-phenyl-pyrazol,
- 4,5-Diamino-1-methyl-3-phenyl-pyrazol,
- 4-Amino-1,3-dimethyl-5-hydrazino-pyrazol,
- 1-Benzyl-4,5-diamino-3-methyl-pyrazol,
- 4,5-Diamino-3-*tert*.-butyl-1-methyl-pyrazol,
- 4,5-Diamino-1-*tert*.-butyl-3-methyl-pyrazol,
- 4,5-Diamino-1-(β-hydroxyethyl)-3-methyl-pyrazol,
- 4,5-Diamino-1-ethyl-3-methyl-pyrazol,
- 4,5-Diamino-1-ethyl-3-(4'-methoxyphenyl)-pyrazol,
- 4,5-Diamino-1-ethyl-3-hydroxymethyl-pyrazol,
- 4,5-Diamino-3-hydroxmethyl-1-methyl-pyrazol,
- 4,5-Diamino-3-hydroxymethyl-1-isopropyl-pyrazol,
- 4,5-Diamino-3-methyl-1-isopropyl-pyrazol,
- 4-Amino-5-(2'-aminoethyl)amino-1,3-dimethyl-pyrazol,
und den Additionssalzen dieser Verbindungen mit einer Säure.

4. Zusammensetzung nach einem der vorhergehenden Ansprüche, dadurch gekennzeichnet, daß das oder die in 3-Stellung substituierte(n) 4,5-Diaminopyrazol(e) der Formel (I) 0,0005 bis 12 Gew.-% des Gesamtgewichts der Färbemittelzusammensetzung ausmachen.

5. Zusammensetzung nach Anspruch 4, dadurch gekennzeichnet, daß das oder die in 3-Stellung substituierte(n) 4,5-Diaminopyrazol(e) der Formel (I) 0,005 bis 6 Gew.-% des Gesamtgewichts der Färbemittelzusammensetzung ausmachen.

6. Zusammensetzung nach einem der vorhergehenden Ansprüche, dadurch gekennzeichnet, daß das zum Färben geeignete Medium (oder der Träger) aus Wasser oder einem Gemisch aus Wasser und mindestens einem organischen Lösungsmittel besteht, das unter den niederen C₁₋₄-Alkoholen, Glycerin, Glykolen und Glykolethern, aromatischen Alkoholen und analogen Produkten und deren Gemischen ausgewählt ist.

7. Zusammensetzung nach einem der vorhergehenden Ansprüche, dadurch gekennzeichnet, daß sie einen pH-Wert im Bereich von 3 bis 12 aufweist.

8. Zusammensetzung nach einem der vorhergehenden Ansprüche, dadurch gekennzeichnet, daß sie mindestens eine zusätzliche Oxidationsbase enthält, die unter den p-Phenylendiaminen, bis-Phenylalkylendiaminen, p-Aminophenolen, o-Aminophenolen und heterocyclischen Basen, die von den in 3-Stellung substituierten 4,5-Diaminopyrazolen der Formel (I) verschieden sind, ausgewählt ist.

9. Zusammensetzung nach Anspruch 8, dadurch gekennzeichnet, daß die zusätzliche(n) Oxidationsbase(n) 0,0005 bis 12 Gew.-% des Gesamtgewichts der Färbemittelzusammensetzung ausmachen.

10. Zusammensetzung nach einem der vorhergehenden Ansprüche, dadurch gekennzeichnet, daß sie mindestens einen Kuppler und/oder mindestens einen Direktfarbstoff enthält.

11. Zusammensetzung nach Anspruch 10, dadurch gekennzeichnet, daß der oder die Kuppler unter den m-Phenylendiaminen, m-Aminophenolen, m-Dihydroxybenzolen und heterocyclischen Kupplern und den Additionssalzen dieser Verbindungen mit einer Säure ausgewählt sind.

12. Zusammensetzung nach Anspruch 10 oder 11, dadurch gekennzeichnet, daß der oder die Kuppler 0,0001 bis 10 Gew.-% des Gesamtgewichts der Färbemittelzusammensetzung ausmachen.

13. Zusammensetzung nach einem der vorhergehenden Ansprüche, dadurch gekennzeichnet, daß die Additionssalze mit einer Säure unter den Hydrochloriden, Hydrobromiden, Sulfaten, Tartaten, Lactaten und Acetaten ausgewählt sind.

14. Verfahren zum Färben von Keratinfasern und insbesondere von menschlichen Keratinfasern, wie dem Haar, dadurch gekennzeichnet, daß auf die Fasern mindestens eine Färbemittelzusammensetzung nach einem der Ansprüche 1 bis 13 während einer Zeitspanne aufgetragen wird, die ausreichend ist, um die gewünschte Färbung entweder an Luft oder mit Hilfe eines Oxidationsmittels zu entwickeln.

15. Verfahren nach Anspruch 14, dadurch gekennzeichnet, daß die Färbung nur in Kontakt mit Luftsauerstoff entwickelt wird.

16. Verfahren nach Anspruch 15, dadurch gekennzeichnet, daß die Färbung nur in Kontakt mit Luftsauerstoff in Gegenwart von Oxidationskatalysatoren entwickelt wird.

17. Verfahren nach Anspruch 16, dadurch gekennzeichnet, daß die Oxidationskatalysatoren Metallsalze sind.

18. Verfahren nach Anspruch 14, dadurch gekennzeichnet, daß die Farbe bei einem sauren, neutralen oder alkalischen pH-Wert mit einem Oxidationsmittel entwickelt wird, das bei der Anwendung der Färbemittelzusammensetzung zugegeben wird oder das in einer oxidierenden Zusammensetzung vorliegt, die gleichzeitig oder getrennt davon anschließend aufgetragen wird.

19. Verfahren nach Anspruch 18, dadurch gekennzeichnet, daß das Oxidationsmittel unter Wasserstoffperoxid, Harnstoffperoxid, Alkalibromaten und Salzen von Persäuren, wie Perboraten und Persulfaten, ausgewählt ist.

20. Vorrichtung mit mehreren Abteilungen oder "Kit" zum Färben mit mehreren Abteilungen, wobei eine erste Abteilung eine Färbemittelzusammensetzung nach einem der Ansprüche 1 bis 13 und eine zweite Abteilung eine oxidierende Zusammensetzung enthält.

21. Neue in 3-Stellung substituierte 4,5-Diamino-pyrazole und die Additionssalze dieser Verbindungen mit einer Säure der folgenden Formel: 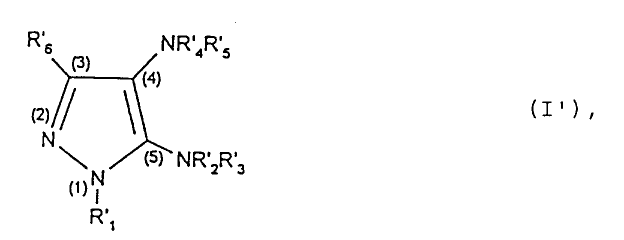 worin R'₁, R'₂, R'₃, R'₄, R'₅ und R'₆ die für die Gruppen R₁, R₂, R₃, R₄, R₅ und R₆ in Formel (I) angegebenen Bedeutungen aufweisen, mit der Maßgabe, daß:
(i) wenn R'₁ Methyl, R'₂, R'₄ und R'₅ gleichzeitig Wasserstoff und R'₃ Wasserstoff oder Methyl bedeuten, R'₆ von Hydroxymethyl, Isobutyloxymethyl, Methoxyethyloxymethyl, Cyclohexyl, Thiophen, Pyridin, Phenyl oder Phenyl, das mit Methyl, Trifluormethyl oder Chlor substituiert ist, verschieden ist;
(ii) wenn R'₁ unsubstituiertes Phenyl und R'₂, R'₃, R'₄ und R'₅ gleichzeitig Wasserstoff bedeuten, R'₆ von unsubstituiertem Phenyl verschieden ist;
(iii) wenn R'₁ unsubstituiertes Phenyl, R'₆ Methyl und R'₂, R'₄ und R'₅ gleichzeitig Wasserstoff bedeuten, R'₃ von Wasserstoff, Methyl oder unsubstituiertem Phenyl verschieden ist;
(iv) wenn R'₁ unsubstituiertes Phenyl, R'₆ Methyl, R'₄ und R'₅ gleichzeitig Wasserstoff und R'₂ Methyl oder Ethyl bedeuten, R'₃ von unsubstituiertem Phenyl verschieden ist;
(v) wenn R'₁ unsubstituiertes Phenyl, R'₆ Methyl und R'₂, R'₃ und R'₅ gleichzeitig Wasserstoff bedeuten, R'₄ von Methyl verschieden ist;
(vi) wenn R'₂, R'₃, R'₄ und R'₅ gleichzeitig Wasserstoff bedeuten und R'₆ Methyl bedeutet, R'₁ von Phenyl verschieden ist, das mit Chlor, Trifluorethyl, Nitro oder Pyridyl substituiert ist;
(vii) wenn R'₁ Wasserstoff, R'₆ Phenyl oder Phenyl, das mit Chlor, Methyl oder Methoxy substituiert ist, und R'₂, R'₄ und R'₅ gleichzeitig Wasserstoff bedeuten, R'₃ von Wasserstoff, C₁₋₄-Alkyl oder unsubstituiertem Phenyl verschieden ist;
(viii) wenn R'₁, R'₂, R'₃, R'₄ und R'₅ gleichzeitig Wasserstoff bedeuten, R'₆ von Methyl verschieden ist;
(ix) wenn R'₁ β-Hydroxyethyl und R'₂, R'₃, R'₄ und R'₅ gleichzeitig Wasserstoff bedeuten, R'₆ von Methyl oder unsubstituiertem Phenyl verschieden ist;
(x) wenn R'₄ Methyl, R'₅ unsubstituiertes Phenyl, R'₂ und R'₃ gleichzeitig Wasserstoff und R'₁ Wasserstoff oder Methyl bedeuten, R'₆ von unsubstituiertem Phenyl oder Phenyl, das mit Methyl, Ethyl, Methoxy oder Chlor substituiert ist, verschieden ist;
(xi) wenn R'₁ *tert*.-Butyl und R'₂, R'₃, R'₄ und R'₅ gleichzeitig Wasserstoff bedeuten, R'₆ von Methyl verschieden ist;
(xii) wenn R'₁ Pyridyl, R'₂, R'₄ und R'₅ gleichzeitig Wasserstoff und R'₃ Wasserstoff oder Methyl bedeuten, R'₆ von Methyl oder unsubstituiertem Phenyl verschieden ist;
(xiii) wenn R'₁ Methyl, Ethyl oder 4-Aminophenyl, R'₂, R'₄ und R'₅ gleichzeitig Wasserstoff und R'₃ Wasserstoff oder unsubstituiertes Phenyl bedeuten, R'₆ von Methyl verschieden ist;
(xiv) wenn R'₁ Isopropyl und R'₂, R'₄ und R'₅ gleichzeitig Wasserstoff bedeuten, mindestens eine der Gruppen R'₃ und R'₆ von Methyl verschieden ist;
(xv) wenn R'₁ Wasserstoff oder unsubstituiertes Phenyl, R'₂, R'₄ und R'₅ gleichzeitig Wasserstoff und R'₃ Benzyl oder Phenyl, das mit Methyl oder Chlor substituiert ist, bedeuten, R'₆ von Methyl oder unsubstituiertem Phenyl verschieden ist.

22. Verbindungen nach Anspruch 21, dadurch gekennzeichnet, daß sie ausgewählt sind unter:
- 1-Benzyl-4,5-Diamino-3-methyl-pyrazol,
- 4,5-Diamino-1-(β-hydroxyethyl)-3-(4'-methoxyphenyl)-pyrazol,
- 4,5-Diamino-1-(β-hydroxyethyl)-3-(4'-methylphenyl)-pyrazol,
- 4,5-Diamino-1-(β-hydroxyethyl)-3-(3'-methylphenyl)-pyrazol,
- 4,5-Diamino-3-methyl-1-isopropyl-pyrazol,
- 4,5-Diamino-3-(4'-methoxyphenyl)-1-isopropyl-pyrazol,
- 4,5-Diamino-1-ethyl-3-methyl-pyrazol,
- 4,5-Diamino-1-ethyl-3-(4'-methoxyphenyl)-pyrazol,
- 4,5-Diamino-3-hydroxymethyl-1-methyl-pyrazol,
- 4,5-Diamino-1-ethyl-3-hydroxymethyl-pyrazol,
- 4,5-Diamino-3-hydroxymethyl-1-isopropyl-pyrazol,
- 4,5-Diamino-3-hydroxymethyl-1-*tert*.-butyl-pyrazol,
- 4,5-Diamino-3-hydroxymethyl-1-phenyl-pyrazol,
- 4,5-Diamino-3-hydroxymethyl-1-(2'-methoxyphenyl)-pyrazol,
- 4,5-Diamino-3-hydroxymethyl-1-(3'-methoxyphenyl)-pyrazol,
- 4,5-Diamino-3-hydroxymethyl-1-(4'-methoxyphenyl)-pyrazol,
- 1-Benzyl-4,5-diamino-3-hydroxymethyl-pyrazol,
- 4,5-Diamino-3-methyl-1-(2'-methoxyphenyl)-pyrazol,
- 4,5-Diamino-3-methyl-1-(3'-methoxyphenyl)-pyrazol,
- 4,5-Diamino-3-methyl-1-(4'-methoxyphenyl)-pyrazol,
- 3-Aminomethyl-4,5-diamino-1-methyl-pyrazol,
- 3-Aminomethyl-4,5-diamino-1-ethyl-pyrazol,
- 3-Aminomethyl-4,5-diamino-1-isopropyl-pyrazol,
- 3-Aminomethyl-4,5-diamino-1-*tert*.-butyl-pyrazol,
- 4,5-Diamino-3-dimethylaminomethyl-1-methyl-pyrazol,
- 4,5-Diamino-3-dimethylaminomethyl-1-ethyl-pyrazol,
- 4,5-Diamino-3-dimethylaminomethyl-1-isopropyl-pyrazol,
- 4,5-Diamino-3-dimethylaminomethyl-1-*tert*.-butyl-pyrazol,
- 4,5-Diamino-3-ethylaminomethyl-1-methyl-pyrazol,
- 4,5-Diamino-3-ethylaminomethyl-1-ethyl-pyrazol,
- 4,5-Diamino-3-ethylaminomethyl-1-isopropyl-pyrazol,
- 4,5-Diamino-3-ethylaminomethyl-1-*tert*.-butyl-pyrazol,
- 4,5-Diamino-3-methylaminomethyl-1-methyl-pyrazol,
- 4,5-Diamino-3-methylaminomethyl-1-isopropyl-pyrazol,
- 4,5-Diamino-1-ethyl-3-methylaminomethyl-pyrazol,
- 1-*tert*.-Butyl-4,5-Diamino-3-methylaminomethyl-pyrazol,
- 4,5-Diamino-3-[(β-hydroxyethyl)aminomethyl]-1-methyl-pyrazol,
- 4,5-Diamino-3-[(β-hydroxyethyl)aminomethyl]-1-isopropylpyrazol,
- 4,5-Diamino-1-ethyl-3-[(β-hydroxymethyl)aminomethyl]-pyrazol,
- 1-*tert*.-Butyl-4,5-Diamino-3-[(β-hydroxyethyl)aminomethyl]pyrazol
- 4-Amino-5-(β-hydroxyethyl)amino-1,3-dimethyl-pyrazol,
- 4-Amino-5-(β-hydroxyethyl)amino-1-isopropyl-3-methyl-pyrazol,
- 4-Amino-5-(β-hydroxyethyl)amino-1-ethyl-3-methyl-pyrazol,
- 4-Amino-5-(β-hydroxyethyl)amino-1-*tert*.-butyl-3-methylpyrazol,
- 4-Amino-5-(β-hydroxyethyl)amino-1-phenyl-3-methyl-pyrazol,
- 4-Amino-5-(β-hydroxyethyl)amino-1-(2'-methoxyphenyl)-3-methyl-pyrazol,
- 4-Amino-5-(β-hydroxyethyl)amino-1-(3'-methoxyphenyl)-3-methyl-pyrazol,
- 4-Amino-5-(β-hydroxyethyl)amino-1-(4'-methoxyphenyl)-3-methyl-pyrazol,
- 4-Amino-5-(β-hydroxyethyl)amino-1-benzyl-3-methyl-pyrazol,
- 4-Amino-1-ethyl-3-methyl-5-methylamino-pyrazol,
- 4-Amino-1-*tert*.-butyl-3-methyl-5-methylamino-pyrazol,
- 1-(4'-Chlorbenzyl)-4,5-Diamino-3-methyl-pyrazol,
- 4,5-Diamino-3-hydroxymethyl-1-isopropyl-pyrazol,
- 4-Amino-1-ethyl-3-methyl-5-methylamino-pyrazol,
- 4-Amino-5-(2'-aminoethyl)amino-1,3-dimethyl-pyrazol,
und den Additionssalzen dieser Verbindungen mit einer Säure.

23. Verbindungen nach Anspruch 22, dadurch gekennzeichnet, daß sie ausgewählt sind unter:
- 1-Benzyl-4,5-Diamino-3-methyl-pyrazol,
- 4,5-Diamino-1-ethyl-3-methyl-pyrazol,
- 4,5-Diamino-1-ethyl-3-(4'-methoxyphenyl)-pyrazol,
- 4,5-Diamino-1-ethyl-3-hydroxymethyl-pyrazol,
- 4,5-Diamino-3-hydroxymethyl-1-methyl-pyrazol,
- 4,5-Diamino-3-hydroxymethyl-1-isopropyl-pyrazol,
- 4,5-Diamino-3-methyl-1-isopropyl-pyrazol,
- 4,5-Amino-5-(2'-aminoethyl)amino-1,3-dimethyl-pyrazol,
und den Additionssalzen dieser Verbindungen mit einer Säure.

24. Verfahren zur Herstellung einer Verbindung der Formel (I') nach einem der Ansprüche 21 bis 23, wobei R'₆ Methyl bedeutet und R'₁ von Wasserstoff verschieden ist, dadurch gekennzeichnet, daß es darin besteht, in einem ersten Schritt ein 3-Aminocrotononitril mit einem einfach substituierten Hydrazin bei einer Temperatur über 90 °C in einem alkoholischen Lösungsmittel umzusetzen, in einem zweiten Schritt das 5-Aminopyrazol in 4-Stellung durch Umsetzung mit einem anorganischen oder organischen Nitrit zu nitrosieren, wobei ein 5-Amino-4-nitroso-pyrazol gebildet wird, das in einem dritten Schritt durch katalytische Hydrierung zu 4,5-Diaminopyrazolen der Formel (I') führt, worin R'₆ Methyl bedeutet und R'₁ von Wasserstoff verschieden ist.

25. Verfahren zur Herstellung einer Verbindung der Formel (I') nach einem der Ansprüche 21 bis 23, wobei R'₆ von Methyl und R'₁ von Wasserstoff verschieden ist, dadurch gekennzeichnet, daß es darin besteht, in einem ersten Schritt ein β-Ketoacetonitril mit einem einfach substituierten Hydrazin bei einer Temperatur im Bereich von 20 bis 150 °C in einem alkoholischen Lösungsmittel umzusetzen, um ein 5-Amino-pyrazol herzustellen, das dann in einem zweiten Schritt in 4-Stellung nitrosiert wird, wobei ein 4-Nitroso-5-amino-pyrazol gebildet wird, das dann in einem dritten Schritt hydriert wird, wodurch 4,5-Diaminopyrazole der Formel (I') gebildet werden, worin R'₆ von Methyl und R'₁ von Wasserstoff verschieden ist.

26. Verfahren zur Herstellung einer Verbindung der Formel (I') nach einem der Ansprüche 21 bis 23, wobei R'₆ eine Gruppe mit starker sterischer Hinderung darstellt, dadurch gekennzeichnet, daß es darin besteht, in einem ersten Schritt ein β-Ketoacetonitril mit einem einfach substituierten Hydrazin umzusetzen, um ein 5-Aminopyrazol herzustellen, das dann in einem zweiten Schritt in 5-Stellung acetyliert wird, um ein 5-Acetylamino-pyrazol zu ergeben, das dann in einem dritten Schritt in 4-Stellung nitriert und in 5-Stellung desacetyliert wird, um ein 5-Amino-4-nitro-pyrazol zu ergeben, das dann in einem vierten Schritt hydriert wird und zu 4,5-Diaminopyrazolen der Formel (I') führt, in welchen R'₆ eine Gruppe mit starker sterischer Hinderung bedeutet.

27. Verfahren zur Herstellung einer Verbindung der Formel (I') nach einem der Ansprüche 21 bis 23, wobei eine der Gruppen R'₂ oder R'₃ von Wasserstoff verschieden ist, dadurch gekennzeichnet, daß es darin besteht, in einem ersten Schritt einen β-Ketoester mit Hydrazin zur Herstellung eines 5-Hydroxy-pyrazols im Gleichgewicht mit seiner tautomeren Form Pyrazol-5-on herzustellen, das dann in einem zweiten Schritt in 4-Stellung nitriert, daraufhin in einem dritten Schritt in 5-Stellung chloriert wird, um ein 5-Chlor-4-nitro-pyrazol herzustellen, das dann in einem vierten Schritt in Gegenwart eines primären Aminsalzes H₂N-R'₃ zu einem 5-Amino-4-nitropyrazol führt, das dann in einem fünften Schritt durch katalytische Hydrierung 4,5-Diaminopyrazole der Formel (I') ergibt, worin einer der Gruppen R'₂ oder R'₃ von Wasserstoff verschieden ist.
